# EUROPEAN PATENT APPLICATION

(11) **EP 4 843 596 A1**
(43) Date of publication of application: **25.11.2026**
(21) Application number: 26196597.4
(22) Date of filing: 23.12.2021
(51) Int. Cl.: A61M 11/00, A61M 16/00, A61M 16/08, A61M 16/14, A61M 16/20, B05B 14/00, B05B 17/00

(54) **HIGH FLOW AEROSOL BLENDING**

(30) Priority: 23.12.2020 US 202063130061 P
(62) Document number(s) of the earlier application(s) in accordance with Art. 76 EPC: 24195408.0 / 4 477 244; 21848078.8 / 4 267 222
(71) Applicant: Vapotherm, Inc., Exeter, NH 03833 (US)
(72) Inventor: LEONARD, Scott A., Exeter, 03833 (US); ADAMS, David, Exeter, 03833 (US); YEH, Kevin, Exeter, 03833 (US); RAO, Ashish, Exeter, 03833 (US); DUNGAN, George, Exeter, 03833 (US); BODWELL, Jesse, Exeter, 03833 (US)
(74) Representative: Pisani, Diana Jean

(57) **Abstract**

Provided herein are devices, systems, and methods for introducing a nebulized medication into breathing gas for respiratory therapy. An apparatus includes a reservoir to contain a volume of medication, a vibrating mesh disposed at a reservoir outlet of the reservoir to receive a flow of the medication from the reservoir and output a flow of nebulized medication, a mixing chamber having a gas inlet to receive a flow of breathing gas from a gas source, and a nebulizer inlet to receive the flow of nebulized medication from the vibrating mesh, where the vibrating mesh is disposed between the nebulizer inlet and the reservoir outlet. The apparatus can include a pressure tap tube or other implement in fluid communication with the mixing chamber and the reservoir to selectively equalize pressure between the mixing chamber and a headspace in the reservoir above the volume of medication.

## Description

### Cross-Reference to Related Applications

This application claims benefit of and priority to U.S. provisional application number 63/130,061, filed December 23, 2020, the contents of which are hereby incorporated herein by reference in their entirety.

### Background:

Delivering nebulized drug therapy though a nasal cannula poses several significant challenges. The most significant problem is rain-out. This occurs when the nebulized particles impact upon a surface and stick to it. During the course of drug therapy, the surface may be the inner walls of the delivery device where the nebulized particles impact and coalesce into larger droplets which are too large to be respirable. The delivery device may include a delivery tube and a patient interface (such as a nasal cannula, for example). The delivery tube provides a conduit through which the breathing gas and the nebulized particles are provided to the patient from a source located away from the patient, and the nasal cannula provides the breathing gas and the nebulized particles from the delivery tube to the nares of the patient for inhalation.

### Summary:

Provided herein are devices, systems, and methods for introducing medication during high flow therapy. To address the problems presented by high pressures associated with high flow rates of breathing gas, various technical solutions are described to address the problem and introduce medication with a high efficiency and reduced rain out.

In one aspect, provided herein is an adaptor for introducing a nebulized medication into breathing gas for respiratory therapy. The adaptor includes a reservoir adapted to contain a volume of medication; a vibrating mesh disposed at a reservoir outlet of the reservoir and adapted to receive a flow of the medication from the reservoir and output a flow of nebulized medication; a mixing chamber having a gas inlet adapted to receive a flow of breathing gas from a gas source; a nebulizer inlet adapted to receive the flow of nebulized medication from the vibrating mesh, the vibrating mesh being disposed between the nebulizer inlet and the reservoir outlet; an internal volume in fluid communication with the gas inlet and the nebulizer inlet and adapted to allow for mixing of the flow of breathing gas and the flow of nebulized medication to produce a flow of mixed gas; and a mixed gas outlet in fluid communication with the internal volume and adapted to output the flow of mixed gas; and a pressure tap tube in fluid communication with the mixing chamber via a first end of the pressure tap tube and the reservoir via a second end of the pressure tap tube, such that a gas in the pressure tap tube bypasses the nebulizer inlet and vibrating mesh to selectively equalize pressure between the mixing chamber and a headspace in the reservoir above the volume of medication.

In some implementations, the second end of the pressure tap tube includes a connector sized to couple to a port of the reservoir in a sealingly manner and adapted to convey gas from the pressure tap tube into the headspace. For example, the connector is a rubber plug having an inner lumen. In some implementations, the adaptor includes a receiver sized to couple to the connector of the second end of the pressure tap tube, the receiver being isolated from the reservoir such that gas does not flow through the pressure tap tube when the connector is coupled to the receiver. In some implementations, the reservoir includes a valve adapted to couple to the second end of the pressure tap tube. For example, the valve is one of a solenoid valve, a diaphragm valve, or a needle valve. In some implementations, the pressure tap tube is integrally formed in a wall connecting the reservoir to the mixing chamber, the wall at least partially defining the nebulizer inlet and positioned adjacent to the vibrating mesh. In some implementations, the reservoir includes a medication inlet adapted to receive a refill flow of medication into the reservoir from a medication source. For example, the medication inlet includes a sealing plug adapted. In some implementations, the pressure tap tube includes a lumen having an internal diameter between 0.01 in and 0.03 in.

In another aspect, provided herein is a method for providing breathing gas with nebulized medication at a high velocity. The method includes the steps of providing a flow of breathing gas at a first flow rate through a mixing chamber from a gas source; adjusting the flow of breathing gas to a second flow rate lower than the first flow rate; providing a flow of nebulized medication into the mixing chamber via a vibrating mesh; mixing the flow of nebulized medication with the flow of breathing gas at the second flow rate such that a flow of mixed gas is output from the mixing chamber; stopping the flow of nebulized medication by halting operation of the vibrating mesh; and adjusting the flow of breathing gas to the first flow rate.

In some implementations, the breathing gas is provided at the second flow rate during a low flow period and at the first flow rate during a high flow period. In some implementations, the low flow period is synchronized with at least an inspiratory phase of breathing of a patient. In some implementations, the high flow period is synchronized with an expiratory phase of breathing of a patient. In some implementations, the low flow period occurs immediately before the inspiratory phase or immediately after the inspiratory phase. In some implementations, the mixing chamber includes a gas inlet having a first valve and a gas outlet having a second valve, wherein the first valve and second valve are closed while the flow of breathing gas is at the first flow rate, and wherein the first valve and second valve are open while the flow of breathing gas is at the second flow rate.

In another aspect, provided herein is an adaptor for introducing a nebulized medication into breathing gas for respiratory therapy. The adaptor includes a gas inlet adapted to receive a flow of breathing gas from a gas source; a first flow path in fluid communication with the gas inlet and adapted to transmit a first portion of the flow of breathing gas; a second flow path in fluid communication with the gas inlet and adapted to transmit a second portion of the flow of breathing gas, the second flow path includes a mixing chamber adapted to receive the second portion of breathing gas and a flow of nebulized medication, such that the second portion of breathing gas and the flow of nebulized medication mix to form a mixed portion of gas that is output by the second flow path; a reservoir adapted to hold a volume of medication; a vibrating mesh disposed between the reservoir and the mixing chamber and adapted to receive medication from the reservoir and output the flow of nebulized medication to a nebulizer inlet of the mixing chamber; and a gas outlet in fluid communication with the first flow path and the second flow path and adapted to combine the first portion of breathing gas and the mixed portion of gas to output a mixed flow of gas; wherein the first flow path and the second flow path are separated by a first valve, such the first valve may close to seal off the mixing chamber from the gas inlet.

In some implementations, when the flow of breathing gas is provided from the gas source at a high pressure the first valve passively closes to seal off the mixing chamber due to a pressure drop across the first valve generated by the high pressure. In some implementations, when the first valve passively opens when the flow of breathing gas is adjusted to a low pressure. For example, the adaptor further includes a controller operatively coupled to the first valve and the gas source and adapted to control the gas source to adjust the flow of breathing gas from a first flow rate to a second flow rate, the second flow rate being higher than the first flow rate; and close the first valve so as to seal off the mixing chamber. In some implementations, the first valve is a diaphragm valve configured to occlude the second flow path in response to a high pressure flow of breathing gas.

In some implementations, the first valve is positioned at a mixing chamber inlet, and further including a second valve positioned at a mixing chamber outlet, such that the mixing chamber is sealed off from the first flow path when the first valve and the second valve are closed.

In another aspect, provided herein is a nebulizer including a reservoir adapted to hold a volume of medication; a vibrating mesh in fluid communication with the reservoir and adapted to receive medication from the reservoir and output nebulized medication opposite the reservoir; and an aperture connecting the reservoir to the vibrating mesh, the aperture having a cross-section defined by a first length and a second length perpendicular to the first length, the second length being shorter than the first length.

In some implementations, the aperture has an obround shape. In some implementations, the cross-section of the aperture promotes air bubbles to move away from the vibrating mesh in a direction parallel to the first length.

In another aspect, provided herein is a nebulizer including a reservoir adapted to hold a volume of medication and having a medication outlet; a vibrating mesh disposed at the medication outlet and adapted to receive medication from the reservoir and output nebulized medication opposite the reservoir; and a pump having a pump outlet disposed within the reservoir for providing medication to the reservoir, the pump outlet being positioned proximally to the vibrating such that medication output from the pump outlet contacts the vibrating mesh.

In some implementations, the pump is one of a syringe pump, a peristaltic pump, a diaphragm pump, a blower, or a bulb. In some implementations, the pump outlet is at least partially contacting the vibrating mesh. In some implementations, the pump delivers medication into the reservoir at a continuous rate. In some implementations, the pump delivers medication into the reservoir in timed pulses.

In another aspect, provided herein is a nebulizer including a reservoir adapted to hold a volume of medication and having a medication outlet; a vibrating mesh disposed at the medication outlet and adapted to receive medication from the reservoir and output nebulized medication opposite the reservoir; and at least one of: a wicking material positioned within the reservoir and contacting the vibrating mesh, the wicking material being porous so as to draw medication towards to the vibrating mesh while allowing gas to pass through the wicking material away from the vibrating mesh; a protrusion extending into the reservoir proximal to the vibrating mesh; or a hydrophilic surface disposed on at least a portion of an inner surface of the reservoir.

In another aspect, provided herein is a system for providing breathing gas and nebulized medication to a patient. The system includes a pressurized source of breathing gas adapted to provide a flow of breathing gas; an adaptor for introducing a nebulized medication into the breathing gas, the adaptor including: a reservoir adapted to contain a volume of medication; a vibrating mesh disposed at a reservoir outlet of the reservoir and adapted to receive a flow of the medication from the reservoir and output a flow of nebulized medication; and a mixing chamber having: a gas inlet coupled to the pressurized source of breathing gas and adapted to receive the flow of breathing gas from the pressurized source; a nebulizer inlet adapted to receive the flow of nebulized medication from the vibrating mesh, the vibrating mesh being disposed between the nebulizer inlet and the reservoir outlet; an internal volume in fluid communication with the gas inlet and the nebulizer inlet and adapted to allow for mixing of the flow of breathing gas and the flow of nebulized medication to produced a flow of mixed gas; and a mixed gas outlet in fluid communication with the internal volume and adapted to output the flow of mixed gas; and a pressure tap tube in fluid communication with the pressurized source and the reservoir, such that a gas in the pressure tap tube bypasses the mixing chamber to pressurize a headspace in the reservoir above the volume of medication.

In some implementations, the pressure tap tube includes a first end coupled to the pressurized source and a second end coupled to the reservoir. In some implementations, the second end is part of a port adapted to also provide a refill flow of medication into the reservoir from a medication source. In some implementations, the system further includes a controller operatively coupled to the pressurized source to control a flow rate of the flow of breathing gas and to the vibrating mesh to control the output of nebulized medication. In some implementations, the pressure tap tube includes a valve operatively coupled to the controller, such that the controller is configured to control a flow rate of gas through the pressure tap tube. In some implementations, the controller is configured to automatically adjust the flow rate of gas through the pressure tap tube based on the flow rate of breathing gas from the pressurized source in order to control a pressure drop across the vibrating mesh to reach a target pressure drop. In some implementations, the system further includes a nasal cannula in fluid communication with the mixed gas outlet and configured to deliver the flow of mixed gas to a patient.

In another aspect, provided herein is a system for providing breathing gas and nebulized medication to a patient. The system includes a pressurized source of breathing gas adapted to provide a flow of breathing gas; an adaptor for introducing a nebulized medication into the breathing gas, the adaptor including: a reservoir adapted to contain a volume of medication; a vibrating mesh disposed at a reservoir outlet of the reservoir and adapted to receive a flow of the medication from the reservoir and output a flow of nebulized medication; and a mixing chamber having: a gas inlet in fluid communication with the pressurized source of breathing gas and adapted to receive the flow of breathing gas from the pressurized source; a nebulizer inlet adapted to receive the flow of nebulized medication from the vibrating mesh, the vibrating mesh being disposed between the nebulizer inlet and the reservoir outlet; an internal volume in fluid communication with the gas inlet and the nebulizer inlet and adapted to allow for mixing of the flow of breathing gas and the flow of nebulized medication to produced a flow of mixed gas; and a mixed gas outlet in fluid communication with the internal volume and adapted to output the flow of mixed gas; and a pressure tap tube having a first end in fluid communication with the reservoir, such that a gas in the pressure tap tube pressurizes a headspace in the reservoir above the volume of medication.

In some implementations, the pressure tap tube has a second end in fluid communication with the pressurized source such that pressurized gas from the pressurized source is transmitted to the headspace. In some implementations, the system further includes a gas tube coupling the pressurized source to the gas inlet of the mixing chamber, and wherein the pressure tap tube has a second end in fluid communication with one of the delivery tube or the mixing chamber, such that a portion of breathing gas is diverted from the delivery tube or mixing chamber to pressurize the headspace.

In some implementations of any of the above adaptors, methods, or systems, the breathing gas is humidified. In some implementations, the breathing gas is provided at a flow rate between 8 LPM and 60 LPM.

### Brief Description of Drawings:

The foregoing and other objects and advantages will be apparent upon consideration of the following detailed description, taken in conjunction with the accompanying drawings, in which like reference characters refer to like parts throughout, and in which:
FIGS. 1A-1D show air bubble formation in a nebulizer due to a pressure differential, according to an illustrative implementation;
FIG. 2 shows an adaptor for introducing nebulized medication into a high flow gas stream with a pressure tap for pressure equalization, according to an illustrative implementation;
FIG. 3 shows an adaptor for introducing nebulized medication into a high flow gas stream with a pressure tap for pressure equalization, according to another illustrative implementation;
FIG. 4 shows an adaptor for introducing nebulized medication into a high flow gas stream with a pressure tap for pressure equalization, according to a further illustrative implementation;
FIG. 5 shows a plot of breathing gas flow rate synchronized with operation of a nebulizer, according to an illustrative implementation;
FIG. 6 shows an adaptor for introducing nebulized medication into a high flow gas stream by diverting a portion of breathing gas into a mixing chamber isolated from a primary gas conduit by one or more valves, according to an illustrative implementation;
FIG. 7A shows an exemplary nebulizer with a oval aperture around a vibrating mesh, according to an illustrative implementation;
FIG. 7B shows an exemplary nebulizer with an obround aperture around a vibrating mesh, according to an illustrative implementation;
FIG. 8 shows a nebulizer having a medication delivery tube with an outlet disposed near a vibrating mesh, according to an illustrative implementation;
FIG. 9 shows an illustrative machine proximate nebulizer according to an embodiment of the present disclosure;
FIG. 10 shows an illustrative cross sectional view of the machine proximate nebulizer of FIG. 9;
FIGS. 11A-11E show illustrative perspective views of an embodiment of a machine proximate nebulizer adaptor according to an embodiment of the present disclosure;
FIG. 12 shows a flowchart of an illustrative method of providing machine proximate nebulization therapy to a patient;
FIG. 13 shows a flowchart of an illustrative method of cleaning the vibrating mesh of a nebulizer attached to the machine proximate nebulizer adaptor of FIGS. 11A-11E; and
FIG. 14 shows a flowchart of an illustrative method of providing continuous respiratory therapy to a patient using the machine proximate nebulizer adaptor of FIGS. 11A-11E.

### Description:

To provide an overall understanding of the assemblies and methods described herein, certain illustrative implementations will be described. Although the implementations and features described herein are specifically described for high flow or high velocity respiratory therapy, it will be understood that all the components and other features outlined below may be combined with one another in any suitable manner and may be adapted and applied to other respiratory therapy systems and devices, including low flow oxygen therapy, continuous positive airway pressure therapy (CPAP), mechanical ventilation, oxygen masks, Venturi masks, and tracheostomy masks.

The term "about," as used herein, should be understood to mean plus or minus 20%. For example, "about 40 m/s" should be understood to mean 40 m/s ± 8 m/s. As used herein, the term "processor" or "computing device" refers to one or more computers, microprocessors, logic devices, servers, or other devices configured with hardware, firmware, and software to carry out one or more of the computerized techniques described herein. Processors and processing devices may also include one or more memory devices for storing inputs, outputs, and data that is currently being processed. As used herein, "user interface" includes, without limitation, any suitable combination of one or more input devices (e.g., keypads, touch screens, trackballs, voice recognition systems, etc.) and/or one or more output devices (e.g., visual displays, speakers, tactile displays, printing devices, etc.). Examples of user devices that may implement an interface include, without limitation, personal computers, laptops, and mobile devices (such as smartphones, blackberries, PDAs, tablet computers, etc.). For example, an interface may be implemented over a web browser or a mobile application installed on the user device.

High velocity nasal cannula (HVNC), high flow nasal cannula (HFNC), CPAP, BiPAP, and other respiratory therapy systems all require elevated air pressure to function. In the case of CPAP and BiPAP, the pressure is the primary mode of operation. In HVNC and HFNC, pressure is needed to move the flow through the vapor transfer element, delivery tubing and through the cannula prongs. Particularly, HVNC requires high pressure to accelerate the flow to high velocity as it passes through the nasal prongs.

It is also beneficial for these therapies to be combined with nebulizer therapies so that the patient can receive medications without interrupting the therapy. The medication is typically aerosolized through the use of a nebulizer, which is connected into the respiratory therapy system by means of an adapter or an auxiliary port.

The provision of medication during the delivery of high velocity nasal insufflation therapy is often a necessary clinical intervention. The ability to deliver the medication without interruption of respiratory or ventilatory support is clinically important. A common source of nebulized medication for inhalation in humans is a vibrating mesh nebulizer (VMN). Such a nebulizer uses a vibrating plate, forcing small droplets through a fine mesh to create an aerosol into a breathable entrained gas stream for delivery to the lungs, in a condition, which will facilitate deposition at the appropriate anatomical target. During continuous nebulization, a stream of medication is typically delivered using a syringe pump to a holding chamber. The medication reservoir is immediately proximate to and communicates to the 'top' of the vibrating mesh. The vibrating mesh is energized and effectuated using an electrical signal derived from an external generator. The medication is passed through the mesh and enters an admixing chamber. There the medication is incorporated into the breathing gas stream.

The provision of HVNC therapy is made possible by the management of breathing gas delivered to the nasal cannula at relatively high pressures, as compared to ambient pressure. The incorporation of the medication through the VMN is made challenging by this high pressure differential. Pressurized gas from the admixing chamber challenges the VMN introduction of aerosolized medication into the high pressure environment and can force gas through the medication into the medication reservoir (*i*.*e*., retrograde to the intended flow of medication to the patient). The introduction of medical gas through the mesh in such a manner can render the mesh ineffective and serve to discontinue or degrade the efficacy of the VMN. A solution which would reduce the relative differential pressure serves to reduce the likelihood of such a retrograde motion of medical gas and reduce the potential of loculated gas 'blocking' the vibrating mesh.

One particular challenge with introducing nebulized medication into these systems is the pressure inherent to them. Some of the nebulizers that may be used with the technologies described in this specification are based on vibrating mesh technology. In these nebulizers, a thin metal plate or plastic dome with microscopic holes is vibrated. The medication is forced through the holes by the vibration; this process nebulizes the medication. When the pressure on the distal or output side of the metal plate or dome is too high, air may be forced back through the holes. The air may coalesce into a bubble that is attached to the surface of the plate or dome. This bubble may prevent the medication from reaching the holes in the plate or dome and effectively stops nebulization. Once the bubble forms, it is easier for more air to push from the distal or output side through the holes and the bubble grows. When the bubble reaches a certain size (depending, e.g., on fluid pressure and/or viscosity), a part of the bubble may break free and float to the surface of the fluid, but a portion will remain attached to the dome, restarting the bubble and allowing more air to pass through. Recovery form this state typically requires stopping the therapy, removing the nebulizer and agitating the nebulizer to cause the entire bubble to detach. Then the therapy may be restarted.

Described in this specification are technologies including systems and methods for introducing a nebulized medication into breathing gas for respiratory therapy. In some implementations, the technologies described in this specification can be used to reduce or eliminate the formation of bubbles in a liquid medication in a reservoir that is included in or attached to a nebulizer.

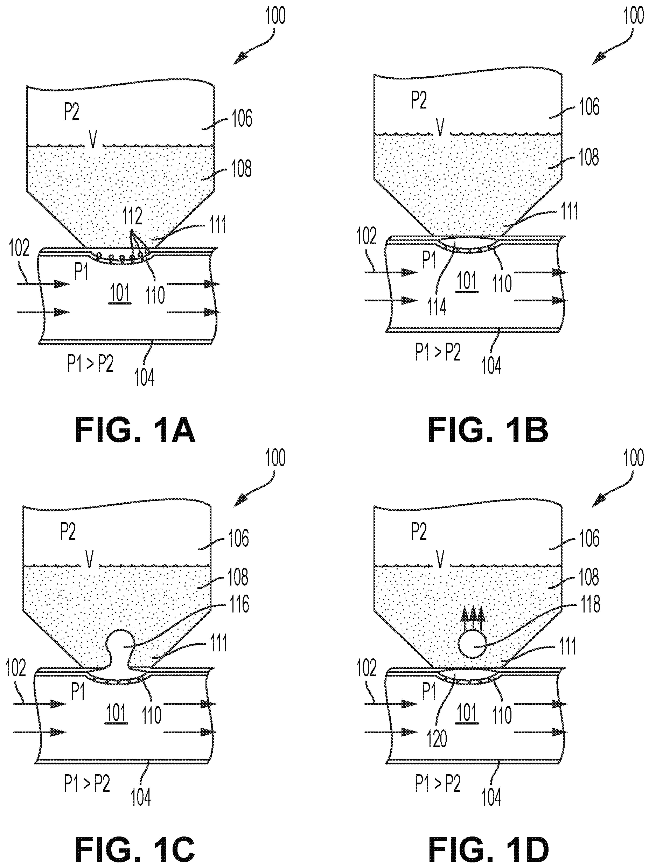

FIGS. 1A-1D show air bubble formation in an illustrative nebulizer 100 due to a pressure differential, according to an illustrative implementation. FIG. 1A-1D show a nebulizer 100 for introducing a medication 108 for nebulization into a flow of breathing gas 102 for respiratory therapy. The nebulizer 100 can include a reservoir 106 adapted to contain a volume of the medication 108 for nebulization, a vibrating mesh 110 disposed in the reservoir outlet 111 of the reservoir 106 to receive a flow of the medication 108 from the reservoir 106 and output a flow of nebulized medication, and a mixing chamber 101 contained with mixing chamber walls 104 for the nebulized medication to mix with the flow of breathing gas 102. The vibrating mesh 110 can include holes (not shown in FIGS. 1A-1D) through which the nebulized medication from the reservoir 106 passes through to mix with the flow of breathing gas 102.

Referring now to FIG. 1A, when a pressure P1 of the mixing chamber 101 is higher than the pressure P2 of the reservoir 106, air can be forced through the holes of the vibrating mesh 108 in a proximal direction from the mixing chamber 101 into the reservoir forming multiple small air bubbles 112. Referring now to FIG. 1B, the small air bubbles 112 formed can coalesce into a single large bubble 114 at the reservoir outlet 111. The single large bubble 114 can block the reservoir outlet 111 and prevent the nebulized medication 108 from mixing with the flow of breathing gas 102 in the mixing chamber 100. Referring now to FIG. 1C, the single large bubble 114 can grow into an enlarged bubble 116. Referring now to FIG. 1D, enlarged bubble 116 can grow until a free bubble 118 breaks away and floats to the surface of the medication 108 in the reservoir 106. A new single large bubble 120 can restart forming an enlarged bubble 116 once the free bubble 118 breaks away, continuing to the block the reservoir outlet 111 and prevent the nebulized medication 108 from mixing with the flow of breathing gas 102 in the mixing chamber 101. The single large bubbles (e.g., bubbles 114 and 120) blocking the reservoir outlet 111 and preventing the nebulized medication 108 from mixing with the flow of breathing gas 102 in the mixing chamber 101 can require removing the nebulizer 100 and agitating the nebulizer 100 to detach the single large bubble 114 before restarting the nebulizer 100. In some implementations, the technologies described in this specification can reduce or prevent the formation of large bubbles (e.g., bubbles 114 and 120), thereby reducing or eliminating the removal and/or restart of the nebulizer.

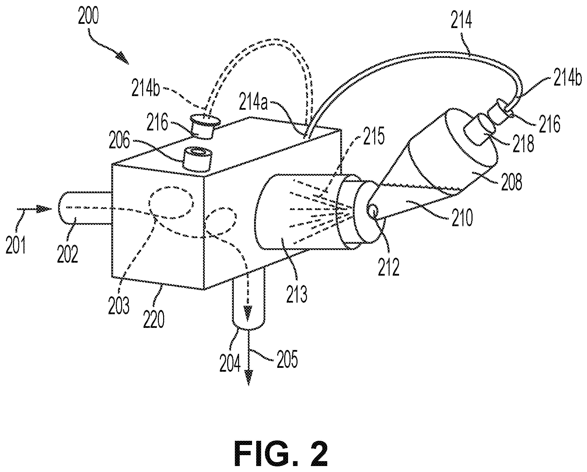

Referring now to FIG. 2, FIG. 2 shows an illustrative adaptor 200 for introducing nebulized medication into a high flow gas stream. The adaptor can include implements for pressure equalization, for example, a pressure tap as described below. The adaptor 200 can include a reservoir 208 to hold a volume of medication 210, a vibrating mesh 212 disposed at a reservoir outlet 211 of the reservoir 208 to receive a flow of the medication 210 from the reservoir 208 and output a flow of nebulized medication 215, and a mixing chamber 220. The mixing chamber 220 can include or can be connected to a gas inlet 202 adapted to receive a flow of breathing gas 201 from a gas source (not shown), e.g., a hospital gas line or a breathing gas generator. The mixing chamber 220 can include or can be connected to a connector 213 adapted to receive the flow of nebulized medication 215 from the vibrating mesh 212. In some implementations, connector 213 can be mounted on a vertical side of mixing chamber 220, e.g., such that vibrating mesh 212 is disposed vertically. As discussed further below, vertical mounting of the mesh 212 can promote removal of bubbles from the mesh. The vibrating mesh 212 can form an interface between the connector 213 and the reservoir outlet 211. For example, vibrating mesh 212 can be integrated into reservoir 208 or connector 213. In some implementations, the vibrating mesh 212 is disposed between reservoir outlet 211 and an inlet of connector 213. The mixing chamber 220 includes an internal volume 203 in fluid communication with the gas inlet 202 and the connector 213 to allow for mixing of the flow of breathing gas 201 and the flow of nebulized medication 215 to produce a flow of mixed gas 205. The mixing chamber 220 can also include or can be connected to a mixed gas outlet 204 in fluid communication with the internal volume 203 and adapted to output the flow of mixed gas 205. The mixed gas 205 can be conveyed to a patient, e.g., via a breathing arrangement including a cannula and tubing in fluid connection to mixed gas outlet 204.

To equalize the pressure between the mixing chamber 220 and the reservoir 208 to prevent the formation of bubbles, the adaptor 200 can include a pressure tap tube 214 to connect the mixing chamber 220 at a first end of the pressure tap tube 214a with the reservoir 208 at a second end of the pressure tap tube 214b. In some implementations, the pressure tap tube 214 also can have a small inner diameter to limit flow of gas while balancing the pressure. In some implementations, the pressure tap tube 214 can have an internal diameter of 0.010 inches to 0.030 inches, for example, a diameter of about 0.010, 0.015, 0.020, 0.025, or 0.030 inches. In some implementations, the pressure tap tube 214 can have an internal diameter of less than 0.010 inches to 0.030 inches. In some implementations, the pressure tap tube 214 can have an internal diameter of more than 0.030 inches. The mixing chamber 220 can also include first receiver 206 to receive the second end of the pressure tap 214b and/or connector 216 when the adaptor 200 is not in use.

In some implementations, the second end of the pressure tap tube 214b includes a connector 216 adapted to couple to a medication inlet 218 of the reservoir 208 to form a pressure seal to convey gas from, e.g., the internal volume 203 of mixing chamber 220 through the pressure tap tube 214 into the reservoir 208. For example, the connector 216 can be a rubber plug having an inner lumen acting as a sealing plug. In some implementations, the connector 216 can be a metal plug or cap, for example, a screw cap. In some implementations, the connector 216 can include a sealing element (e.g., an O-ring). In some implementations, the second end of the pressure tap tube 214b and/or connector 216 can include one or more valves or ports, e.g., to connect one or more additional tubes, e.g. to allow addition of medication while plug 216 is disposed in medication inlet 218. In some implementations, the medication inlet 216 can include a valve (not shown) adapted to couple to the second end of the pressure tap tube 214b. For example, the valve can be one of a solenoid valve, a diaphragm valve, or a needle valve. In some implementations, the adaptor 200 includes a second receiver (not shown) sized to couple to the connector 216 of the second end of the pressure tap tube 214b such that the second receiver is isolated from the reservoir 208 such that gas does not flow through the pressure tap tube 214 when the connector 216 is coupled to the second receiver.

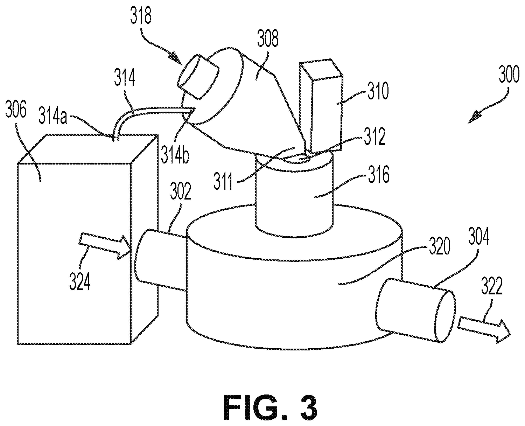

Referring now to FIG. 3, FIG. 3 shows an illustrative adaptor 300 for introducing nebulized medication into a high flow gas stream. The adaptor can include implements for pressure equalization, for example, a pressure tap as described below. The adaptor 300 can include a reservoir 308 to hold a volume of medication (not shown), a vibrating mesh 312 disposed at a reservoir outlet 311 of the reservoir 308 to receive a flow of the medication from the reservoir 308 and output a flow of nebulized medication, an electrical connector 310 to connect the adaptor 300 to an external signal generator (not shown) and a mixing chamber 320. The mixing chamber 320 can include or can be connected to a gas inlet 302 adapted to receive a flow of humidified breathing gas 324 from a gas source 306 e.g., a hospital gas line or a breathing gas generator. The mixing chamber 320 can include or can be connected to a connector 316 adapted to receive a flow of nebulized medication from the vibrating mesh 312. In some implementations, a connector 316 can be mounted horizontally, e.g., mounted on a horizontal plane of mixing chamber 320, e.g., as shown in FIG. 3. In some implementations, a connector 316 can be mounted vertically, e.g., mounted on a vertical plane of mixing chamber 320, e.g., as shown for adaptor 200 in FIG. 2. The vibrating mesh 312 can form an interface between the connector 316 and the reservoir outlet 311. For example, vibrating mesh 312 can be integrated into reservoir 308 or connector 316. In some implementations, the vibrating mesh 312 is disposed between reservoir outlet 311 and an inlet of connector 316. Further, the vibrating mesh 312 can be disposed between the connector 316 and the reservoir outlet 311. The mixing chamber 320 allows for mixing of the flow of breathing gas 324 and the flow of nebulized medication (not shown) to produce a flow of mixed gas 322. The mixing chamber 320 can also include a mixed gas outlet 304 in fluid communication adapted to output the flow of mixed gas 322. The mixed gas 322 can be conveyed to a patient, e.g., via a breathing arrangement including a cannula and tubing in fluid connection to mixed gas outlet 304.

The adaptor 300 presented in FIG. 3 can include a pressure tap tube 314 configured to connect the gas source 306 and the reservoir 308 with a first end of the pressure tap tube 314a and a second end of the pressure tap tube 314b, where the first end of the pressure tab tube 314a can be disposed on or in the gas source 306 and the second end of the pressure tab tube 314b can be disposed on or in the reservoir 308. The pressure tap tube 314 can have a small inner diameter to limit flow of gas while balancing the pressure. In some implementations, the pressure tap tube 314 can have a diameter of 0.010 inches to 0.030 inches, for example, a diameter of about 0.010, 0.015, 0.020, 0.025, or 0.030 inches. In some implementations, the pressure tap tube 314 can have an internal diameter of less than 0.010 inches to 0.030 inches. In some implementations, the pressure tap tube 314 can have an internal diameter of more than 0.030 inches. In some implementations, the second end of pressure tab tube 314b is part of a medication inlet 318 adapted to also provide a refill flow of medication into the reservoir 308 from a medication source (not shown in the figure).

The humidified breathing gas 324 can be maintained at a pressure P1, for example, in breathing gas source 306. The mixing chamber 320 maintains a gas pressure P2. The first end of the pressure tap tube 314a can be connected breathing gas source 306. In some implementations, the second end of the pressure tap tube 314b can be directly connected to the reservoir 308, separate from a medication inlet 318 of the reservoir 308. In some implementations, the second end of the pressure tap tube 314b can be connected to medication inlet 318 of the reservoir 308. Without wishing to be bound by theory, P2 is less than or equal to pressure P1, which can minimize pressure differential across the vibrating mesh 312, thereby reducing the formation of bubbles and allowing the adaptor 300 to function under various pressure regimes with different flow rates, cannula sizes, and viscosity of the medication. In some implementations, the pressure P1 can be derived a gas stream for a patient's therapy (e.g. a patient gas line) or from a separate gas source (not shown). In some implementations, the adapter 300 presented in FIG.3 can include a controller (not shown) operatively coupled to the gas source 306 to control a flow rate of the flow of breathing gas 324. In some implementations, the controller can be connected to the vibrating mesh 312 to control the output of nebulized medication. In some implementations, the pressure tap tube 314 can include one or more valves operatively coupled to the controller, such that the controller can control a flow rate of breathing gas 324 through the pressure tap tube 314. In some implementations, the flow rate of the breathing gas 324 through the pressure tap tube 314 is adjusted automatically based on (e.g., solely based on) the flow rate of breathing gas 324 to control a pressure drop across the vibrating mesh 312, e.g., to reach a target pressure drop. For example, the adaptor 300 can be configured such that an increase in pressure or flow rate through gas inlet 302 causes an increase of flow through pressure tap tube 314. In some implementations, the controller can (automatically) adjust the flow rate of the breathing gas 324 through the pressure tap tube 314 (e.g., using one or more valves operatively coupled to the controller) based on the flow rate of breathing gas 324 in order to control a pressure drop across the vibrating mesh 312 to reach a target pressure drop. In some implementations, adaptor 300 can include one or more sensors, for example, one or more pressure sensors, one or more flow velocity or flow rate sensors, or one or more temperature sensors, or a combination thereof. In some implementations, the controller can adjust the flow rate of the breathing gas 324 through the pressure tap tube 314 (e.g., using one or more valves operatively coupled to the controller) based on one or more measurements obtained from the one or more sensors, for example, from a sensor measuring the flow rate of breathing gas 324, in order to control a pressure drop across the vibrating mesh 312 to reach a target pressure drop.

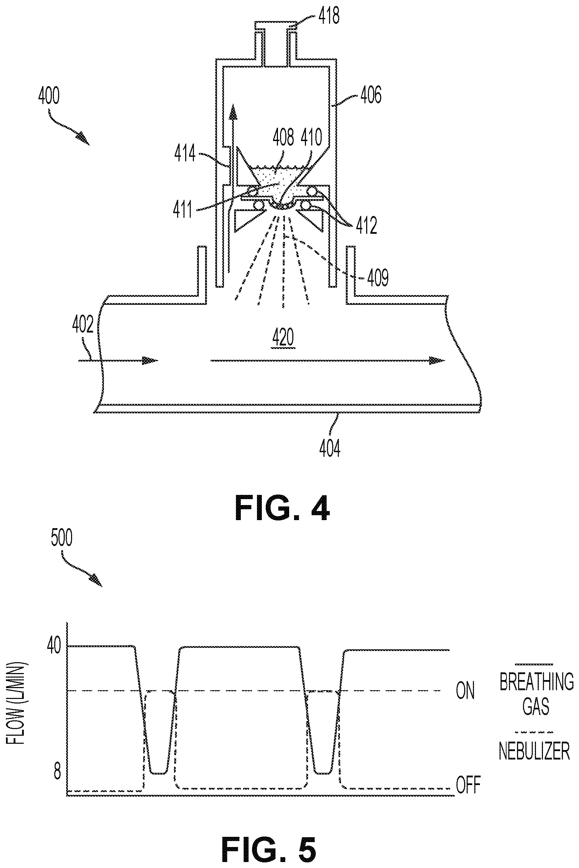

Referring now to FIG. 4, FIG.4 presents an illustrative example implementation of an adaptor 400 for introducing nebulized medication into a gas stream with a pressure tap for pressure equalization. Adaptor 400 and its features can be used in combination with the systems and methods for delivering a breathing gas described in this specification. The adaptor 400 can include a reservoir 406 configured to hold a volume of medication 408, a vibrating mesh 410 disposed at a reservoir outlet 411 of the reservoir 406 configured to receive a flow of the medication 408 from the reservoir 406. The reservoir outlet 411 can output the flow of nebulized medication 409 into a mixing chamber 420 defined by mixing chamber walls 404 to mix with a flow of humidified breathing gas 402 from a gas source (not show). The gas source can provide the flow of the humidified breathing gas 402 at a pressure P1. The mixing chamber 420 can allow for mixing of the flow of breathing gas 402 and the flow of nebulized medication 408 to produce a flow of mixed gas.

In some implementations, the adaptor 400 can include an integral pressure tap tube 414. Integral pressure tap tube 414 is integrated into the body of reservoir 406, which allows for a compact design of the adaptor without the need for external tubing. Pressure tap tube 414 is fluidically connected to the mixing chamber 420 and the reservoir 406 to maintain a pressure P2 inside the mixing chamber 420, where the pressure inside the reservoir 406 is the same or less than pressure P1. To maintain the pressure inside the reservoir 406, the reservoir 406 can include O-rings 412 disposed between the vibrating mesh 410 and features of the reservoir 406 holding the mesh at the reservoir outlet 411 to create a pressure seal.

Referring to FIG. 5, FIG.5 presents a plot 500 of breathing gas flow rate synchronized with operation of a nebulizer, which includes an adaptor, in reference to all implementations of the adaptor discussed in this specification. For example, referring to the implementation of the adaptor 300 presented in FIG. 3, the gas source 306 can provide a flow of breathing gas 324 at a first flow rate through the mixing chamber 320 from the gas source 306 when the nebulizer is switched off. For example, the first flow rate of the breathing gas can be between 30 L/Min and 60 L/Min, for example, 40 L/Min as shown in FIG. 5. In the instances when the nebulizer is turned on (and provides nebulized medication at a nebulizer flow rate of, e.g., between 0.01 L/Min and 30 L/Min, e.g., 0.01, 0.1, 0.5, 1, 10, or 20 L/Min), the gas source 306 can provide the breathing gas 324 at a second flow rate, through the mixing chamber 320 for mixing the flow of nebulized medication from the reservoir 308 with the flow of breathing gas 324. The second flow rate of breathing gas is lower than the first flow rate. The second flow rate can be between 5 L/Min and 15 L/Min, e.g., 8 L/Min as shown in Fig. 5. When the flow rate is decreased, the pressure in the mixing chamber 320 decreases and a higher percentage of nebulized medication reaches the patient. For example, the percentage of nebulized medication reaching the patient can be 200% to 1000% higher when synchronized with the second lower flow rate compared with the first higher flow rate. In some implementations, a nebulizer duty cycle of 10% to 50% at the second lower flow rate can deliver the same amount of nebulized medication when compared to continuous nebulizer operation at the first flow rate. In some implementations, the second flow rate can be higher than or equal to the first flow rate to meet the needs of a patient.

In some implementations, an adaptor as described in this specification, for example adaptor 300 presented in FIG. 3, can include a sensor to detect breathing. For example, a gas source (e.g., the gas source 306) can provide breathing gas 324 at the second lower flow rate to emit the medication just before or during the inspiratory phase of breathing. This allows the first higher flow rate to flush CO2 during expiratory phase, and a greater percentage of medication to be delivered when the patient is actively inhaling. In some implementations, the gas source 306 adjusts the flow rate of the breathing gas 324 from the second lower flow rate to the first higher flow rate when the nebulizer is turned off. In some implementations, the breathing gas is provided at the second flow rate during a low flow period and at the first flow rate during a high flow period. In some implementations, the low flow period is synchronized with at least an inspiratory phase of breathing of a patient. In some implementations, the first flow rate is synchronized with an expiratory phase of breathing of a patient. In some implementations, the gas source 306 is configured to provide the breathing gas 324 at the second lower flow rate immediately before the inspiratory phase or immediately after the inspiratory phase. In some implementations, a gas inlet of an adaptor (e.g., the gas inlet 302 of the mixing chamber 320) can include a first valve and a mixed gas outlet of an adaptor (e.g., the mixed gas outlet 324) can include a second valve, for example, as described below. In some implementations, the first valve and second valve are closed while the flow of breathing gas 324 is at the first flow rate, and the first valve and second valve are open while the flow of breathing gas 324 is at the second flow rate.

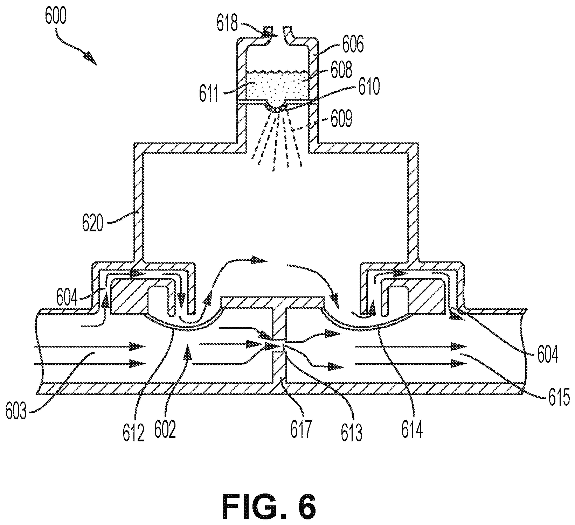

Referring now to FIG. 6, FIG. 6 shows an illustrative example implementation of an adaptor for introducing nebulized medication into a high flow gas stream by diverting a portion of breathing gas into a mixing chamber isolated from a primary gas conduit by one or more valves. Adaptor 600 and its features can be used in combination with the systems and methods for delivering a breathing gas described in this specification. The adaptor 600 can include a reservoir 606 to hold a volume of medication 608, a vibrating mesh 610 disposed at a reservoir outlet 611 of the reservoir 606 to receive a flow of the medication 608 from the reservoir 606 and output a flow of nebulized medication 609, and a mixing chamber 620. The reservoir includes a medication inlet 618, which may include an opening to the environment an/or a valve. The adaptor can also include a gas inlet 603 adapted to receive a flow of breathing gas 602 from a gas source (not shown), a first flow path 613 in fluid communication with the gas inlet 603 adapted to transmit a first portion of the flow of the breathing gas 602, a second flow path 604 in fluid communication with the gas inlet 603 adapted to transmit a second portion of the flow of the breathing gas 602, the second flow path 604 extending in part through mixing chamber 620. The mixing chamber 620 can receive the second portion of breathing gas 602 and the flow of nebulized medication 609, such that the second portion of breathing gas 602 and the flow of nebulized medication 609 mix to form a mixed portion of gas that is output by the second flow path 604. The first flow path 613 and the second flow path 604 can be separated by a first valve 612, where the first valve 612 can be adapted seal off the mixing chamber 620 from the gas inlet 603. The adaptor can combine the first portion of breathing gas 602 and the mixed portion of gas to output a mixed flow of gas. The first flow path 613 and the second flow path 604 can be separated by a first valve 612, such that the first valve may close to seal off the mixing chamber from the gas inlet. In some implementations, the adaptor 600 can include a gas outlet 615 downstream of gas inlet 603. In some implementations, gas outlet 615 and gas inlet 603 are fluidically separated by an orifice plate 617. The adaptor 600 can also include a second valve 614 (e.g., downstream of the first valve 612) at or near gas outlet 615 to separate the first flow path 613 and the second flow path 604, such that the mixing chamber 620 is sealed off from the first flow path 613 when the first valve 612 and the second valve 614 are closed.

The flow of breathing gas 602 can be provided from the gas source (not shown) at a high pressure, which causes the first valve 612 to passively close to seal off the mixing chamber 620 due to a pressure drop across the first valve 612 generated by the high pressure in gas inlet 603. In some implementations, second valve 614 can passively close to seal off the mixing chamber 620 due to a pressure drop across the second valve 614 generated by the high pressure in gas outlet 615. In some implementations, the first valve 612 (and/or second valve 614) passively opens when the flow of breathing gas 602 is adjusted to a low pressure (in gas inlet 603 and/or gas outlet 614, respectively). In some implementations, the adaptor 600 can include a controller (not shown) operatively coupled to the first valve 612 and/or the gas source. In some implementations, the controller is operatively coupled to second valve 614. The controller can control the gas source to adjust the flow rate of breathing gas 602 from a first higher flow rate to a second lower flow rate than the first flow rate and causing the first valve 612 to open, allowing breathing gas to enter the mixing chamber 620. Lowering the flow rate can cause the second valve 614 to open, allowing breathing gas to exit the mixing chamber 620 and to enter gas outlet 615. In some implementations, the first valve 612 is a diaphragm valve adapted to occlude the second flow path 604 in response to a high pressure flow of breathing gas 602. In some implementations, the second valve 614 is a diaphragm valve adapted to occlude the second flow path 604 in response to a high pressure flow of breathing gas 602. In some implementations, the first valve 612 is positioned at a mixing chamber inlet, and a second valve 614 is positioned at a mixing chamber outlet, such that the mixing chamber is sealed off from the first flow path when the first valve and the second valve are closed.

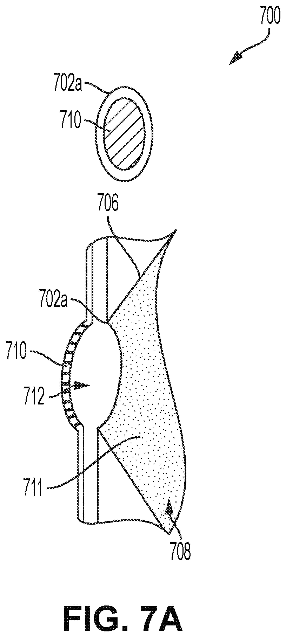

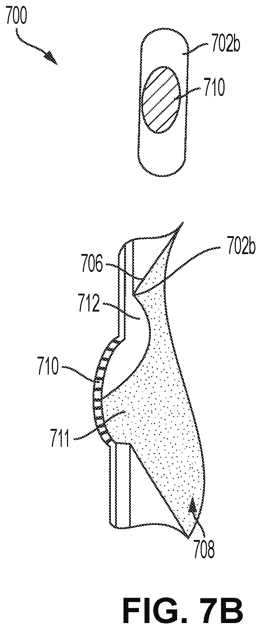

FIG. 7A shows an exemplary nebulizer with an oval aperture around a vibrating mesh, according to an illustrative implementation and FIG. 7B shows an exemplary nebulizer with an obround aperture around a vibrating mesh, according to an illustrative implementation. Nebulizer 700 and its features can be used in combination with the systems and methods for delivering a breathing gas described in this specification. Referring now to FIGS. 7A and 7B, the nebulizer 700 can include a reservoir 706 configured to hold a volume of medication 708, a vibrating mesh 710 disposed at a reservoir outlet 711 of the reservoir 706 to receive a flow of the medication 708 from the reservoir 706 and output a flow of nebulized medication 708 into a mixing chamber (not shown). The nebulizer 700 can include an aperture 702 at reservoir outlet 711 connecting the reservoir 706 to the vibrating mesh 710. Aperture 702 includes a cross-section defined by a first length and a second length perpendicular to the first length. In some implementations, the second length is shorter than the first length. In some implementations, such as shown in FIG. 7A, the aperture 702a has an oval (for example, circular) shape. In some implementations, such as shown in FIG. 7B, the aperture 702b has an obround shape. In some implementations, the aperture 702 can be configured to be shaped as an oval, rectangle, ellipse, or any irregular shape with a dimension along a first axis that is greater a dimension along a second axis perpendicular to the first axis. In some implementations, for example, as shown in FIG. 7B, where the cross-section of the aperture 702b is obround and the mesh is disposed vertically, the shape of the aperture can promote air bubbles 712 to move away (e.g., rise) from the vibrating mesh in a direction parallel to the vertical first length due to bubble buoyancy. Without wishing to be bound by theory, when a bubble forms in a circular aperture, it is evenly connected to the circular opening by surface tension. An obround or other elongated shape, such as an oval, rectangle, ellipse, or an irregular shape with one (vertical) dimension that is greater than the dimension on a perpendicular (horizontal) axis may be used instead. When the longer dimension is oriented vertically, with the shorter dimension oriented horizontally, the bubble will tend to rise toward the upper end of the aperture 702b and may allow the medication to re-contact the mesh.

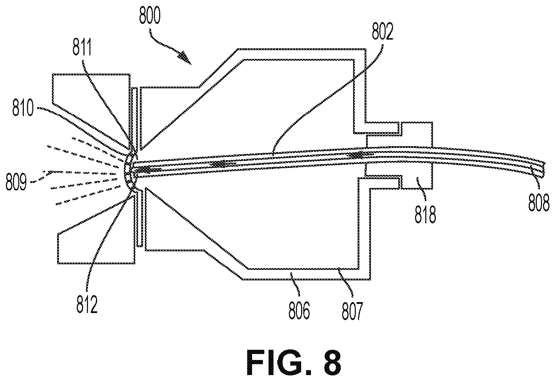

Referring now to FIG. 8, FIG. 8 presents an exemplary nebulizer according to an illustrative implementation. Nebulizer 800 and its features can be used in combination with the systems and methods for delivering a breathing gas described in this specification. Nebulizer 800 includes a reservoir 806 adapted to hold a volume of medication 808, a vibrating mesh 810 disposed at the reservoir outlet 811 to receive medication 808 from the reservoir 806 and output nebulized medication 809 e.g., to a mixing chamber (not shown). The nebulizer 800 can include a pump (not shown) having a pump outlet 802 disposed through a medication inlet 818 and within the reservoir 806 for providing medication 808 to the reservoir 806 where a distal end of the pump outlet 802 can be positioned proximally to the vibrating mesh 810 such that medication 808 from the pump outlet 802 contacts the vibrating mesh 810. The pump outlet 802 can be or can include a tube onto which the medication 808 drips on drop at a time and runs down to the vibrating mesh 810. The pump outlet 802 can contact the vibrating mesh 810 at a contact point 812 at the distal end for the medication 808 to directly interact the vibration mesh 810 and push away any bubbles formed (not shown).

A pump used with the adaptors described in this specification can be one of a syringe pump, a peristaltic pump, a diaphragm pump, a blower, or a bulb. In some implementations, a pump outlet, for example, the pump outlet 802, is at least partially contacting the vibrating mesh, for example, mesh 810. In some implementations, the pump delivers medication into a reservoir at a continuous rate. In some implementations, the pump delivers medication into the reservoir in timed pulses.

The nebulizers described in this specification can include a wicking material positioned within the reservoir and contacting the vibrating mesh. The wicking material can be porous material so as to draw medication towards the vibrating mesh while allowing breathing gas to pass through the wicking material away from the vibrating mesh. The nebulizers can include a protrusion extending into the reservoir proximal to the vibrating mesh. The reservoir can also be configured to include a hydrophilic surface disposed on at least a portion of an inner surface of the reservoir.

In another aspect, provided herein is a method for providing breathing gas and nebulized medication to a patient in reference to the nebulizers and adaptors described in this specification. For example, referring to the illustrative adaptor 300 for introducing nebulized medication into a high flow gas stream described in FIG. 3, the method includes flowing form the pressurized gas source 306, a breathing gas 324 through breathing gas inlet 302 into a mixing chamber 320 and introducing the nebulized medication into the breathing gas 324. The method includes providing the reservoir 308 adapted to contain a volume of medication and a vibrating mesh 312 disposed at a reservoir outlet 311 of the reservoir 308, and flowing a volume of the nebulized medication from the reservoir 308 through the mesh and outputing a flow of nebulized medication to the mixing chamber 320. The method includes mixing the flow of breathing gas 324 and the nebulized medication to produce a flow of mixed gas 322. The method includes flowing the flow of mixed gas 322 out of a mixed gas outlet 304 in fluid communication with mixing chamber 320. The method also includes equalizing the pressure of the reservoir 308 and the mixing chamber 320 using a pressure tap tube 314 in fluid communication with the pressurized gas source 306 and the reservoir 308, such that the breathing gas 324 in the pressure tap tube 314 bypasses the mixing chamber 320 to pressurize the reservoir 308. In some implementations, the method includes delivering the mixed gas 322 to a patient, e.g., thorough a tubing and/or a cannula coupled to mixed gas outlet 304.

The adaptors described in this specification (e.g., adaptors 200, 300, 400, or 800, or any combination thereof) can be placed in several locations along the therapy flow path of a breathing apparatus to introduce nebulized medication to the breathing gas being delivered to the patient. In some implementations, the adaptor can be introduced to the supply of breathing gas before humidification of the breathing gas in the gas source, for example, at or immediately downstream of a gas source, for example, gas source 306. Introducing the medication before humidifying the breathing gas presents certain advantages including collecting the rain-out before the breathing gas with the nebulized medication can be sent to the patient. The orientation of the adaptor can be fixed at any desired angle. This configuration may be used for continuous delivery of nebulized medication over an extended time.

In some implementations, an adaptor as described in this specification (e.g., adaptors 200, 300, 400, or 800, or any combination thereof) can be introduced to the supply of breathing gas after the humidification of the breathing gas in the gas source, for example, downstream of a humidifier. In this configuration, a collection reservoir for collecting the rain-out is needed to prevent the rain-out from being sent to the patient. This collection reservoir must occasionally be emptied, but the collection reservoir may be sized to minimize the frequency at which it must be emptied. The orientation of the adaptor can also be fixed at any desired angle in this configuration, and this configuration can also be used for continuous delivery of medication over an extended time.

In some implementations, an adaptor as described in this specification (e.g., adaptors 200, 300, 400, or 800, or any combination thereof) can be introduced to the supply of breathing gas proximate to the patient. In some implementations, the adaptor is disposed in the nasal cannula, which is disposed to provide the breathing gas to the patient. In some implementations, the adaptor is disposed immediately upstream and/or attached to the nasal cannula, which is disposed to provide the breathing gas to the patient. In this configuration, the pressure at this location is lower than at the machine proximate positions. In this configuration, the adaptor may be subject to movement along with the movement of the patient and can require a caregiver holding the adaptor throughout the therapy. In this configuration, rain-out can build up and requires periodic emptying. This configuration may be suited for acute delivery of medication over a short period of time.

An adaptor as described in this specification (e.g., adaptors 200, 300, 400, or 800, or any combination thereof) can include or can be operatively coupled to a computing device. A computing device may be implemented for performing any of the processes described herein. Each of the components of these systems may be implemented on one or more computing devices. In certain aspects, a plurality of the components of these systems may be included within one computing device. In certain implementations, a component and a storage device may be implemented across several computing devices.

The computing device includes at least one communications interface unit, an input/output controller, system memory, and one or more data storage devices. The system memory includes at least one random access memory (RAM) and at least one read-only memory (ROM). All of these elements are in communication with a central processing unit (CPU) to facilitate the operation of the computing device. The computing device may be configured in many different ways. For example, the computing device may be a conventional standalone computer or alternatively, the functions of computing device may be distributed across multiple computer systems and architectures. The computing device is linked, via network or local network, to other servers or systems.

The computing device may be configured in a distributed architecture, wherein databases and processors are housed in separate units or locations. Some units perform primary processing functions and contain at a minimum a general controller or a processor and a system memory. In distributed architecture implementations, each of these units may be attached via the communications interface unit to a communications hub or port (not shown) that serves as a primary communication link with other servers, client or user computers and other related devices. The communications hub or port may have minimal processing capability itself, serving primarily as a communications router. A variety of communications protocols may be part of the system, including, but not limited to: Ethernet, SAP, SASTM, ATP, BLUETOOTHTM, GSM and TCP/IP.

The CPU includes a processor, such as one or more conventional microprocessors and one or more supplementary co-processors such as math co-processors for offloading workload from the CPU. The CPU is in communication with the communications interface unit and the input/output controller, through which the CPU communicates with other devices such as other servers, user terminals, displays, or devices, such as the nebulizers, respiratory systems, or adaptors described herein. The communications interface unit and the input/output controller may include multiple communication channels for simultaneous communication with, for example, other processors, servers or client terminals.

The CPU is also in communication with the data storage device. The data storage device may include an appropriate combination of magnetic, optical or semiconductor memory, and may include, for example, RAM, ROM, flash drive, an optical disc such as a compact disc or a hard disk or drive. The CPU and the data storage device each may be, for example, located entirely within a single computer or other computing device; or connected to each other by a communication medium, such as a USB port, serial port cable, a coaxial cable, an Ethernet cable, a telephone line, a radio frequency transceiver or other similar wireless or wired medium or combination of the foregoing. For example, the CPU may be connected to the data storage device via the communications interface unit. The CPU may be configured to perform one or more particular processing functions.

The data storage device may store, for example, (i) an operating system for the computing device; (ii) one or more applications (e.g., computer program code or a computer program product) adapted to direct the CPU in accordance with the systems and methods described here, and particularly in accordance with the processes described in detail with regard to the CPU; or (iii) database(s) adapted to store information that may be utilized to store information required by the program.

The operating system and applications may be stored, for example, in a compressed, an uncompiled and an encrypted format, and may include computer program code. The instructions of the program may be read into a main memory of the processor from a computer-readable medium other than the data storage device, such as from the ROM or from the RAM. While execution of sequences of instructions in the program causes the CPU to perform the process steps described herein, hard-wired circuitry may be used in place of, or in combination with, software instructions for implementation of the processes of the present invention. Thus, the systems and methods described are not limited to any specific combination of hardware and software.

Suitable computer program code may be provided for performing one or more functions described herein. The program also may include program elements such as an operating system, a database management system and "device drivers" that allow the processor to interface with computer peripheral devices (e.g., a video display, a keyboard, a computer mouse, etc.) via the input/output controller.

The term "computer-readable medium" as used herein refers to any non-transitory medium that provides or participates in providing instructions to the processor of the computing device (or any other processor of a device described herein) for execution. Such a medium may take many forms, including but not limited to, non-volatile media and volatile media. Non-volatile media include, for example, optical, magnetic, or opto-magnetic disks, or integrated circuit memory, such as flash memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, punch cards, paper tape, any other physical medium with patterns of holes, a RAM, a PROM, an EPROM or EEPROM (electronically erasable programmable read-only memory), a FLASH-EEPROM, any other memory chip or cartridge, or any other non-transitory medium from which a computer can read.

Various forms of computer readable media may be involved in carrying one or more sequences of one or more instructions to the CPU (or any other processor of a device described herein) for execution. For example, the instructions may initially be borne on a magnetic disk of a remote computer (not shown). The remote computer can load the instructions into its dynamic memory and send the instructions over an Ethernet connection, cable line, or even telephone line using a modem. A communications device local to a computing device (e.g., a server) can receive the data on the respective communications line and place the data on a system bus for the processor. The system bus carries the data to main memory, from which the processor retrieves and executes the instructions. The instructions received by main memory may optionally be stored in memory either before or after execution by the processor. In addition, instructions may be received via a communication port as electrical, electromagnetic or optical signals, which are exemplary forms of wireless communications or data streams that carry various types of information.

Delivering nebulized drug therapy though a nasal cannula poses several significant challenges. The most significant problem is rain-out. This occurs when the nebulized particles impact upon a surface and stick to it. During the course of drug therapy, the surface may be the inner walls of the delivery device where the nebulized particles impact and coalesce into larger droplets which are too large to be respirable. The delivery device may include a delivery tube and a patient interface (such as a nasal cannula, for example). The deliver tube provides a conduit through which the breathing gas and the nebulized particles are provided to the patient from a source located away from the patient, and the nasal cannula provides the breathing gas and the nebulized particles from the delivery tube to the nares of the patient for inhalation.

When nebulized particles are contained in a flow of breathing gas, any change in flow direction or turbulence can cause particles to impact a surface. This is exacerbated by high velocity flows encountered in HFT where the change in direction is more abrupt. In such situations, the gas is able to change direction, but the inertia of the particles causes them to move toward the outside of any turn and impact the walls of either the delivery tube or nasal cannula. Nasal cannulas are inherently small in cross section and therefore result in gases flowing through them at high velocity. Further, nasal cannulas have a number of direction changes incorporated into their configuration. Thus any nebulized particles that have not rained-out in the delivery tube may impact the inner walls of the nasal cannula thereby contributing further to rain-out. If the rain-out is sent to the patient, the large drops are clinically ineffective and irritating to the patient.

A high proportion of nebulized drugs rain-out in the delivery device during therapy. As an example, by using a cannula manufactured by Vapotherm, Inc. of Exeter, New Hampshire, USA, during nebulizer based drug therapy, approximately 75% of the nebulized drug will rain-out in the cannula. Sending a large quantity of drug, e.g. 10mL/hr to 20mL/hr, will therefore result in a corresponding large amount of rain-out, e.g. 7.5mL/hr to 15mL/hr, at the cannula due to accumulation of rain-out in the delivery tube and rain-out in the cannula. Large amounts of rain-out will cause discomfort for the patient due to the buildup of condensed medicament or drug in the cannula. Using a cannula manufactured by Vapotherm, Inc. of Exeter, New Hampshire, USA, for example, it has been shown that by removing 70 to 90% of the particles from the output of the nebulizer prior to delivery results in a significant reduction in rain-out at the cannula while delivering sufficient amounts of the drug to the patient for the required treatment.

It is therefore advantageous to remove excess medicament from the output of the nebulizer before the nebulized particles reach the nasal cannula. In one option, excess rain-out can be removed from the flow of breathing gas at the patient proximate end of the delivery tube just before connection to the nasal cannula. However this requires a reservoir or rain-out trap near the patient to collect the liquid medicament, which may be uncomfortable and cumbersome for the patient. The rain-out trap must also be held at a particular angle to avoid a bolus of drug being delivered to the patient, which can be challenging when the patient moves during treatment. In another option, excess rain-out can be removed at the machine proximate end of the delivery tube. However in HFT systems the high velocity flow is very likely to cause re-entrainment of the removed excess rain-out back into the flow of breathing gas. The removed excess rain-out would also need to be trapped and disposed of. Considering that 70 to 90% of the nebulized drug is removed to reduce the rain-out in the system as mentioned above, disposal of such a large proportion of the nebulized drug would mean that more of the drug would be necessary to maintain the efficacy of the treatment. This could incur significant cost if the drug is expensive.

Disclosed herein are approaches for addressing various of the problems and shortcomings of the state of the art, as identified above. More particularly, disclosed herein are systems and methods for providing respiratory therapy to a patient. According to a first embodiment of the present disclosure, there is provided a system comprising a nebulizer operable to aerosolize a medicament where the medicament is transformed from a liquid into an aerosol of medicament particles. The system also comprises a cylindrical mixing chamber having an inner surface, an inlet port, an outlet port, an aerosol port in fluid communication with the nebulizer, and a drainage port. The inlet port is configured to receive a flow of breathing gas from a source of breathing gas. The outlet port has a first end and a second end, the second end external to the mixing chamber and configured to interfit with a delivery tube for delivery of the flow of breathing gas to the patient. The mixing chamber is configured to receive a flow of aerosol from the nebulizer via the aerosol port, entrain aerosol into the flow of breathing gas, and deliver the breathing gas entrained with aerosol to the outlet port. The system further comprises an impacting cap in fluid communication with the mixing chamber, the impacting cap having a sloped inner surface and forming a settling volume (or settling space) with the mixing chamber. The settling volume may have a diameter that is larger a diameter of the inlet port and a diameter of the aerosol port. The mixing chamber may be configured to receive the aerosol in the settling volume and cause a portion of the aerosol to coalesce into droplets in or on any of the settling volume, the inner wall of the mixing chamber, and the sloped inner surface of the impacting cap. The inner wall of the mixing chamber and the sloped inner surface of the impacting cap may be configured to direct rain-out (also referred to as liquid or condensate in the present disclosure) resulting from the droplets to the drainage port. The system includes a recirculation tube adapted to return the rain-out to the nebulizer.

The system causes a portion of the nebulized medicament particles to coalesce on the inner walls of the mixing chamber and the inner walls of the impacting cap. These droplets result in rain-out which collects at the drainage port of the mixing chamber. The nebulized medicament remaining in the mixing chamber is entrained into the flow of breathing gas and delivered to the patient. The system therefore encourages rain-out to occur before entrainment of the remaining nebulized particles into the flow of breathing gas. In this manner, the breathing gas entrained with the remaining nebulized medicament is less likely to rain-out in the delivery tube when it is transported to the patient. This increases the efficacy of the treatment as the remaining nebulized particles that are entrained in the flow of breathing gas are less likely to rain-out. This a significantly higher proportion of the nebulized particles are inhaled by the patient. Rain-out is less likely in the delivery tube after the breathing gas entrained with the remaining nebulized medicament leaves the mixing chamber. Thus no bolus of liquid medicament builds up in the delivery tube or nasal cannula, thereby reducing patient discomfort during treatment. The recirculation tube delivers the rain-out to the nebulizer to be reused. This minimizes wastage of the therapy drug and reduces the cost of such therapy especially when expensive drugs are used.

According to a second embodiment of the present disclosure, there is provided a nebulizer adaptor for providing continuous aerosol therapy to a patient. The adaptor comprises a cylindrical mixing chamber having an inlet port, an outlet port, an aerosol port in fluid communication with the nebulizer, and a drainage port. The inlet port is configured to receive a flow of breathing gas from a source of breathing gas. The outlet port has a first end and a second end, the second end external to the mixing chamber and configured to interfit with a delivery tube for delivery of the flow of breathing gas to the patient. The mixing chamber is configured to receive a flow of aerosolized medicament from a vibrating mesh nebulizer via the aerosol port, entrain aerosol into the flow of breathing gas, and deliver the breathing gas entrained with aerosol to the outlet port. The adaptor also comprises an impacting cap in fluid communication with the mixing chamber, the impacting cap having a sloped inner surface and forming a settling volume with the mixing chamber. The mixing chamber has a diameter that is larger than a diameter of the inlet port and a diameter of the aerosol port, and is configured to receive the aerosol in the settling volume and cause a first portion of the aerosol to coalesce into droplets in or on any of the settling volume, the inner wall of the mixing chamber, and the sloped inner surface of the impacting cap. Further, the inner wall of the mixing chamber and the sloped inner surface of the impacting cap being configured to direct rain-out resulting from the droplets toward the drainage port.

In some implementations, the impacting cap may be located above the inlet port, outlet port, aerosol port and drainage port. The drainage port may be located below the inlet port, outlet port, aerosol port and impacting cap. This ensures that the rain-out is directed towards the drainage port and is distanced from the outlet port so that the rain-out does not get re-entrained into the flow of breathing gas. In certain implementations, the first end of the outlet port may be orthogonally oriented to the inlet port. The first end of the outlet port may be vertically spaced from the inlet port of the mixing chamber. The first end of the outlet port may extend into the mixing chamber. Such configurations encourage the nebulized particles and breathing gas to move up the mixing chamber, inducing rain-out along the way.

In certain implementations, the inlet port and the outlet port may be in fluid communication such that the breathing gas entrained with aerosol may be provided to the delivery tube via the second end of the outlet port. The outlet port may be arranged orthogonally to the inlet port.

In some implementations, the impacting cap may be cone shaped. The walls of the impacting cap may be symmetrical about the outlet port. The impacting cap may also be contiguous with the mixing chamber. This encourages the rain-out to slide down the inner walls of the impacting cap towards the drainage port. This reduces the risk of the rain-out from becoming re-entrained into the flow of breathing gas at the outlet port. This also minimizes the possibility of the rain-out flowing directly into the outlet port.

In some implementations, the nebulizer may comprise a reservoir containing the medicament. The rain-out from the recirculation tube may be dumped and stored in the nebulizer reservoir. Further, the nebulizer may comprise an aerosol chamber. This allows the stream of nebulized particles leaving the nebulizer outlet to reach an equilibrium velocity before being introduced into the mixing chamber. The aerosol chamber may comprise a baffle, that may be adjustable, to control the stream of nebulized particles (and hence drug delivery rate) into the mixing chamber.

In certain implementations, the recirculation tube may comprise an auxiliary port for the introduction of additional or different medicament to the nebulizer. This additional or different medicament gets dumped into the nebulizer, which subsequently becomes nebulized and delivered to the patient. In some embodiments, the mixing chamber may comprise a collection compartment adjacent to the drainage port to enable the rain-out to collect prior to being drained by the drainage port. A stopcock may also be coupled to the drainage port or the recirculation tube to enable the rain-out to be stored in the collection compartment prior to being recycled to the nebulizer. This may be beneficial when introducing different medicament into the nebulizer via the auxiliary port.

In further implementations, the drainage port may be connected to a receptacle via a siphon to drain excess rain-out away from the nebulizer. This allows a swap out of medicament as the patient's therapy requirements change. The siphon may have a valve for ease of control. The drainage port may be coupled to a flow restrictor to control a flow rate of the recirculated rain-out.

In some implementations, the mesh is configured to vibrate in order to aerosolize the medicament. In certain implementations, a second portion of the aerosol coalesces into droplets on a surface of the mesh during vibration. In other implementations, a conduit fluidically connects the inlet port and the aerosol port, the conduit in fluid communication with the mixing chamber for the delivery of breathing gas from the inlet port to the mixing chamber. In further implementations, the conduit is aligned along a direction that is normal to the surface of the mesh as the conduit fluidically connects the inlet port to the aerosol port. In some implementations, the conduit directs the breathing gas received by the inlet port to impinge upon the surface of the mesh as it flows along the conduit to the mixing chamber. In certain implementations, the breathing gas removes the droplets from the surface of the mesh upon impingement.

In other implementations, the nebulizer is removable from the aerosol port of the mixing chamber. In some implementations, a removable cap is configured to seal the aerosol port after removal of the nebulizer. In certain implementations, the conduit directs the flow of breathing gas to the mixing chamber and output port for delivery to the patient after the aerosol port is sealed.

In some implementations, the medicament may comprise at least one of: bronchodilators, surfactants and antibiotics. The medicament may comprise at least one of: Albuterol (Ventolin), Salbutamol (Proventil), Levosalbutamol/Levalbuterol (Xopenex), Curosurf (Chiesi Pharmaceuticals), Alveofact (Boehringer Ingelheim), Survanta (Abbott Laboratories), Exosurf (Glaxo Wellcome), Surfaxin (Discovery Laboratories), macrolides, erythromycin, clarithromycin, azithromycin, glycopeptides, vancomycin, teicoplanin, oxazoldinone, quinupristin/dalfopristen, aminoglycosides, gentamicin, tobramycin, amikacin, streptomycin, netilmicin, quinolones, ciprofloxacin, ofloxacin, levofloxacin, tetracyclines, oxytetracycline, doxycycline, minocycline, cotrimoxazole, colistin, imepinim, and meripenim.

According to a third embodiment of the present disclosure, there is provided a method for providing respiratory therapy to a patient. The method comprises providing a flow of breathing gas to a settling volume defined by a mixing chamber and an impacting cap in fluid communication with the mixing chamber, generating an aerosol from a nebulizer containing a medicament, and providing the aerosol to the settling volume. The method also comprises coalescing a first portion of the aerosol into droplets in or on any of the settling volume, an inner wall of the mixing chamber and a sloped inner surface of the impacting cap, and recirculating rain-out resulting from the droplets to the nebulizer. Further, the method comprises entraining a remaining portion of the aerosol into the flow of breathing gas within the settling volume, and delivering the breathing gas entrained with aerosol from the settling volume to the patient. In this manner, the breathing gas entrained with the remaining nebulized medicament is less likely to rain-out in a delivery tube when it is transported to the patient. This increases the efficacy of the treatment as the remaining nebulized particles that are entrained in the flow of breathing gas are less likely to rain-out. This a significantly higher proportion of the nebulized particles are inhaled by the patient. Rain-out is less likely in the delivery tube after the breathing gas entrained with the remaining nebulized medicament leaves the mixing chamber. Thus no bolus of liquid medicament builds up in the delivery tube or nasal cannula, thereby reducing patient discomfort during treatment. A recirculation tube delivers the rain-out to the nebulizer to be reused. This minimizes wastage of the therapy drug and reduces the cost of such therapy especially when expensive drugs are used.

In some implementations, the method may further comprise reducing a flow velocity of the breathing gas and the aerosol upon entry to the mixing chamber. The enables the aerosol particles to coalesce into droplets within the settling volume. This also allows the aerosol particles to coalesce on the inner wall of the mixing chamber and/or the sloped inner surface of the impacting cap. In some implementations, the method may further comprise storing the rain-out in a reservoir contained within the nebulizer. In this manner, the rain-out from the recirculation tube may be dumped and stored in the nebulizer reservoir for further treatment/use. In certain implementations, the method may comprise storing the aerosol in an aerosol chamber prior to providing the aerosol to the mixing chamber. This allows the stream of nebulized particles leaving the nebulizer outlet to reach an equilibrium velocity before being introduced into the mixing chamber. In further implementations, the method may comprise adjusting the amount of aerosol delivered to the mixing chamber with a baffle. This allows for control of the stream of nebulized particles (and hence drug delivery rate) into the mixing chamber.

In some implementations, the method may comprise providing additional drug to the nebulizer using an auxiliary port connected to the recirculation tube. This additional or different medicament gets dumped into the nebulizer, which subsequently becomes nebulized and delivered to the patient. In certain implementations, the method may additional comprise controlling the flow rate of the recirculating rain-out with a flow restrictor coupled to the drainage port. In further implementations, the method comprises removing excess rain-out from the recirculation tube via a siphon connected to a drug receptacle. This allows for excess rain-out to be drained away from the nebulizer, thereby allowing for a swap out of medicament as the patient's therapy requirements change. In other implementations, the method comprises storing the rain-out in a collection compartment of the mixing chamber prior to recirculating the rain-out to the nebulizer.

In further implementations, the method comprises providing the flow of breathing gas to the settling volume via a conduit that directs the breathing gas to impinge upon a surface of the mesh along a direction that is normal to the surface of the mesh. In other implementations, the method comprises flowing the breathing gas at a higher rate than that used for delivery to the patient, and removing aerosol that has coalesced into droplets on the surface of the mesh by the impingement of the breathing gas on the surface of the mesh. In certain implementations, the method comprises removing the nebulizer from the aerosol port, sealing the aerosol port, and directing the flow of breathing gas to the settling volume for delivery to the patient.

In some implementations, the medicament may comprise at least one of: bronchodilators, surfactants and antibiotics. The medicament may comprise at least one of: Albuterol (Ventolin), Salbutamol (Proventil), Levosalbutamol/Levalbuterol (Xopenex), Curosurf (Chiesi Pharmaceuticals), Alveofact (Boehringer Ingelheim), Survanta (Abbott Laboratories), Exosurf (Glaxo Wellcome), Surfaxin (Discovery Laboratories), macrolides, erythromycin, clarithromycin, azithromycin, glycopeptides, vancomycin, teicoplanin, oxazoldinone, quinupristin/dalfopristen, aminoglycosides, gentamicin, tobramycin, amikacin, streptomycin, netilmicin, quinolones, ciprofloxacin, ofloxacin, levofloxacin, tetracyclines, oxytetracycline, doxycycline, minocycline, cotrimoxazole, colistin, imepinim, and meripenim.

Variations and modifications will occur to those of skill in the art after reviewing this disclosure. The disclosed features may be implemented, in any combination and subcombination (including multiple dependent combinations and subcombinations), with one or more other features described herein. The various features described or illustrated above, including any components thereof, may be combined or integrated in other systems. Moreover, certain features may be omitted or not implemented.

Disclosed herein are systems and methods that provide respiratory therapy to a patient using a mixing chamber in fluid communication with an impacting cap, the mixing chamber and impacting cap creating a settling volume, the mixing chamber being attached to a source of breathing gas. The mixing chamber is also coupled to a nebulizer and is provided with a stream of nebulized medicament therefrom. The mixing chamber and impacting cap is described below and mixes a flow of breathing gas and the stream of nebulized medicament so as to cause a portion of the nebulized medicament particles to impact on the inner walls of the mixing chamber and impacting cap. The aerosol particles coalesce in or on any of the settling volume, the inner wall of the mixing chamber, and the sloped inner surface of the impacting cap, and form rain-out, which collects at a drainage port of the mixing chamber. A recirculation tube is attached to the drainage port and is adapted to deliver the rain-out to the nebulizer to be reused. This increases the efficiency of the system as wastage of the therapy drug is kept to a minimum. This may have an impact on the cost of such therapy for expensive drugs.

The nebulized medicament remaining in the mixing chamber is entrained into the flow of breathing gas and delivered to the patient. In the system of the present disclosure, the mixing chamber and impacting cap encourages rain-out to occur before entrainment of the remaining nebulized particles into the flow of breathing gas. The flow velocity of the aerosol particles is reduced upon entry to the settling volume. The flow of breathing gas into the mixing chamber causes the aerosol particles to me moved upwards in the settling volume, thereby affording the a portion of the aerosol particles to impact the inner wall of the mixing chamber and the sloped inner surface of the impacting cap. In this manner, the breathing gas entrained with the nebulized medicament is made up of remaining aerosol particles and is less likely to rain-out in the delivery tube when it is transported to the patient. This means a significantly higher proportion of the nebulized particles that reach the patient connector (e.g. nasal cannula) are inhaled by the patient. Rain-out is less likely in the delivery tube after the breathing gas entrained with the remaining nebulized medicament leaves the mixing chamber and so a rain-out trap is not required at the end of the delivery tube.

The impacting cap may be located above the inlet port, outlet port, aerosol port and drainage port. The drainage port may be located below the inlet port, outlet port, aerosol port and impacting cap. This ensures that the rain-out is directed towards the drainage port and is distanced from the outlet port so that the rain-out does not get re-entrained into the flow of breathing gas. The first end of the outlet port may be orthogonally oriented to the inlet port. The first end of the outlet port may be vertically spaced from the inlet port of the mixing chamber. The first end of the outlet port may extend into the mixing chamber. Such configurations encourage the nebulized particles and breathing gas to move up the mixing chamber, inducing rain-out along the way.

The impacting cap may be cone shaped. The inner walls of the impacting cap may be symmetrical about the outlet port. The impacting cap may also be contiguous with the mixing chamber. This encourages the rain-out to slide down the inner walls of the impacting cap towards the drainage port. This reduces the risk of the rain-out from becoming re-entrained into the flow of breathing gas at the outlet port. This also minimizes the possibility of the rain-out flowing directly into the outlet port.

The nebulizer may comprise an aerosol chamber. This allows the stream of nebulized particles leaving the nebulizer outlet to reach an equilibrium velocity before being introduced into the mixing chamber. The aerosol chamber may comprise a baffle, that may be adjustable, to control the stream of nebulized particles (and hence drug delivery rate) into the mixing chamber.

The recirculation tube may comprise an auxiliary port for the introduction of additional or different medicament to the nebulizer. This additional or different medicament gets dumped into the nebulizer, which subsequently becomes nebulized and delivered to the patient. The mixing chamber may comprise a collection compartment adjacent to the drainage port to enable the rain-out to collect prior to being drained by the drainage port. A stopcock may also be coupled to the drainage port or the recirculation tube to enable the condensate to be stored in the collection compartment prior to being recycled to the nebulizer. This may be beneficial when introducing different medicament into the nebulizer via the auxiliary port. The drainage port may be connected to a receptacle via a siphon to drain excess condensate away from the nebulizer. This allows a swap out of medicament as the patient's therapy requirements change. The siphon may have a valve for ease of control.

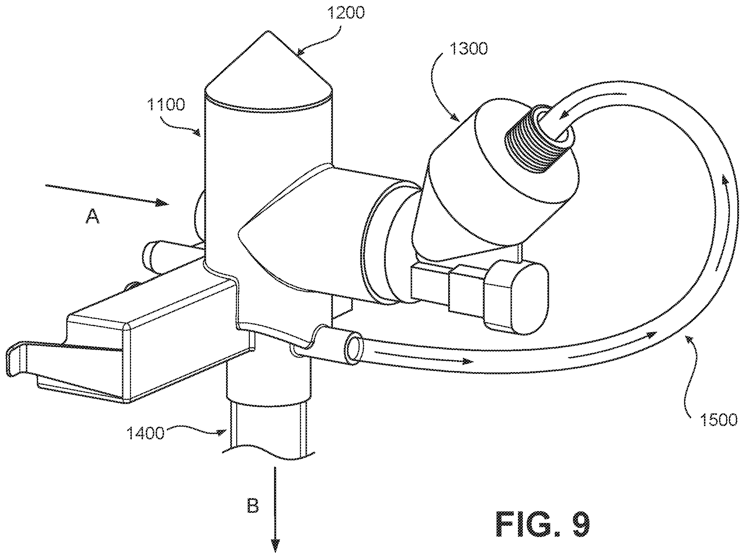

FIG. 9 shows an illustrative system for providing respiratory therapy to a patient. The system comprises a mixing chamber 1100 connected to an impacting cap 1200 and a nebulizer 1300. The mixing chamber 1100 is in fluid communication with the impacting cap 1200, and together form a settling volume. The nebulizer 1300 is operable to provide a stream of nebulized particles to the mixing chamber 1100. A source of breathing gas is attached to the mixing chamber 1100 so as to provide the mixing chamber 1100 with a flow of breathing gas, as indicated by the arrow 'A' in FIG. 9. The breathing gas may be heated and humidified to reduce patient discomfort. The stream of nebulized particles generated by the nebulizer 1300 is mixed with the flow of breathing gas in the mixing chamber 1100. Here a portion of the nebulized particles collide with the inner walls of the impacting cap 1200 and coalesce into droplets to form rain-out. The remaining nebulized particles are entrained in the flow of breathing gas and are fed into a delivery tube 1400 connected to the mixing chamber 1100, as indicated by the arrow 'B' in FIG. 9.

The delivery tube 1400 is in fluid communication with a nasal cannula attached the patient such that the remaining nebulized particles entrained in the flow of breathing gas are provided to the patient thereby providing respiratory therapy to the patient. Exemplary nasal cannulas that can be used in conjunction with the present disclosure are described in U.S. Patent Application Nos. 13/665,1100, 15/199,158 and 62/555,945, the contents of which are hereby incorporated by reference in their entirety. Alternatively, any nasal cannula can be used with the system and method of the present disclosure. The rain-out in the mixing chamber 1100 is returned to the nebulizer 1300 via a recirculating tube 1500 to be re-used as necessary. In certain embodiments, the mixing chamber 1100 is located at the machine end of the delivery tube 1400, away from the patient and proximate to the source of breathing gas.

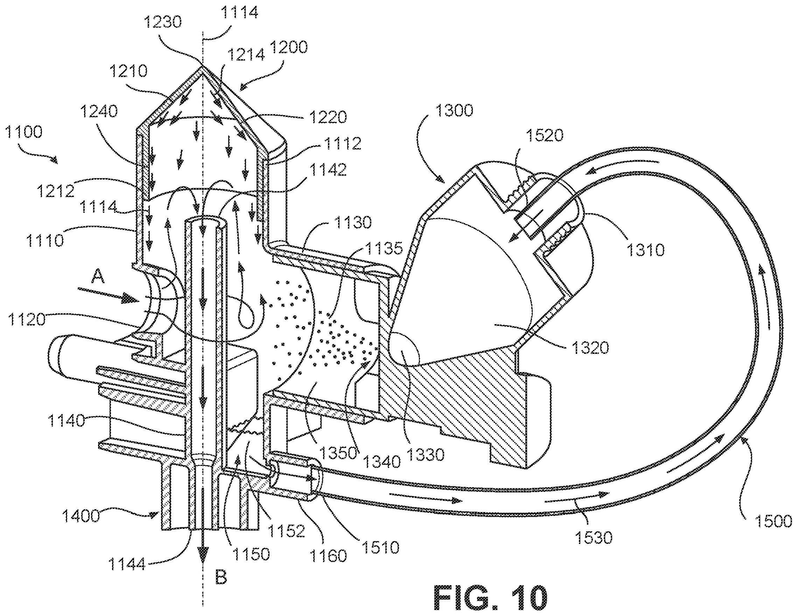

FIG. 10 shows an illustrative detailed cross-section of the system in FIG. 9. As can be seen from FIG. 10, the mixing chamber 1100 comprises a body section 1110 having an opening 1112 to which the impacting cap 1200 is connected. The mixing chamber 1100 is in fluid communication with the impacting cap 1200, and together form a settling volume. The mixing chamber 1100 also comprises an inlet port 1120, an aerosol port 1130, an outlet port 1140 and a drainage port 1160. The mixing chamber 1100 may also comprise a collection chamber 1150 for collecting rain-out that develops in the mixing chamber 1100. The inlet port 1120 is connected to the source of breathing gas via a feed tube to provide the mixing chamber 1100 with the flow of breathing gas. In some embodiments the inlet port 1120 may be cylindrical. The inlet port 1120 may have a diameter that is smaller than the diameter of the body section 1110 of the mixing chamber 1100. This causes the flow velocity of breathing gas to be reduced upon entering the settling volume defined by the mixing chamber 1100 and the impacting cap 1200. In certain embodiments, the inlet port 1120 may be arranged above the drainage port 1160. While FIG. 10 illustrates the body section 1110 of the mixing chamber 1100 as being cylindrical having an axis 1114 and a diameter, in some embodiments, the body section 1110 can be of any shape having substantially vertical walls without deviating from the scope of the present disclosure. In certain embodiments, the diameter of the mixing chamber 1100 may be significantly larger than that of the feed tube through which the source of breathing gas is provided. In certain embodiments, the cross-section of the mixing chamber 1100 may be significantly larger than the cross-section of the inlet port 1120 and the aerosol port 1130. This reduces the flow velocity of the breathing gas and the nebulized particles upon entry to the settling volume defined by the mixing chamber 1100 and the impacting cap 1200.

The source of breathing gas may be configured to provide, for example, breathing gas at flow rates between 1 and 8 lpm for infants, between 5 and 20 lpm for pediatric patients, or up to 40 lpm for adults. In some embodiments, the breathing gas is heated and humidified to increase patient comfort. Suitable sources of heated and humidified gas will be known to one of ordinary skill in the art. For example, the source may be the Vapotherm Flowrest System, Vapotherm Careflow System, Precision Flow unit, or the Vapotherm 2000i, all of which are provided by Vapotherm, Inc. of Exeter, New Hampshire, USA. Other suitable sources of breathing gas will be known to one of ordinary skill in the art from the description herein.

The impacting cap 1200 comprises sloped portions 1210, 1220 and a coupling portion 1240. The sloped portions 1210, 1220 form a surface on which the nebulized particles from nebulizer 1300 collide when in the settling volume. In some embodiments, the sloped portions 1210, 1220 may be symmetrical about the axis 1114 of the mixing chamber 1100. In certain embodiments in which the body section 1110 of the mixing chamber 1100 is a cylinder, the sloped portions 1210, 1220 form a cone with an apex 1230. The cone is symmetrical about the axis 1114 of the mixing chamber 1100 and therefore aligns the apex 1230 with the axis 1114. In some embodiments the body section 1110 may be of any suitable shape that is symmetrical about the axis 1114 and provides at least one surface for the nebulizer particles to collide with. Coupling portion 1240 may be a cylinder with an axis that aligns with axis 1114, as shown in FIG. 10. Coupling portion 1240 may have a diameter that is marginally smaller than the diameter of the body section 1110 thereby facilitating an interference fit between the impacting cap 1200 and the mixing chamber 1100. In other embodiments, the body section 1110 of the mixing chamber 1100 may have an internal thread that mates with an exterior thread formed on the coupling portion 1240 of the impacting cap 1200. The edge 1212 of the coupling portion 1240 may be beveled so as to provide a smooth transition for droplets that coalesce on the inner walls of the impacting cap 1200 to slide down the inner wall of the mixing chamber 1100. In some embodiments, the mixing chamber 1100 and the impacting cap 1200 may be contiguous. In certain embodiments, the mixing chamber 1100 and the impacting cap 1200 may be integrally formed. In some embodiments, the impacting cap 1200 is located above the inlet port 1120, the outlet port 1140, the aerosol port 1130 and the drainage port 1160.

The outlet port 1140 comprises a first end 1142 and a second end 1144, and is arranged such that the first end 1142 extends into the body section 1110 of the mixing chamber 1100, and the second end 1144 of the outlet port 1140 is located external to the mixing chamber 1100. The second end 1144 of the outlet port 1140 is adapted to interfit with a first end of the delivery tube 1400 so as to transport the nebulized particles entrained into the flow of breathing gas from the mixing chamber 1100 to the patient. A second end of the delivery tube 1400 is attached to a nasal cannula attached to the patient such that the nebulized particles can be inhaled by the patient. In certain embodiments, the outlet port 1140 is cylindrically shaped with an axis that aligns with axis 1114 of the mixing chamber. In this arrangement, the outlet port 1140 is orthogonally oriented to the inlet port 1120 of the mixing chamber 1100. In certain embodiments, the first end 1142 of the outlet port 1140 is arranged such that it is located above the inlet port 1120, the aerosol port 1130, the collection chamber 1150 and the drainage port 1160. In certain embodiments, the outlet port 1142 may be vertically spaced from the inlet port 1120 of the mixing chamber 1100. In some embodiments, the outlet port 1140 extends into the settling volume defined by the mixing chamber 1100 and the impacting cap 1200.

Nebulizer 1300 comprises an input port 1310 that provides liquid medicament to a reservoir 1320 for storage prior to being nebulized. Nebulizer 1300 further comprise an aerosol generating mechanism 1330 that is in fluid contact with the liquid medicament in reservoir 1320. The aerosol generating mechanism 1330 is in fluid communication with an outlet port 1340. In certain embodiments, the aerosol generating mechanism 1300 may comprise a vibrating mesh that aerosolizes the liquid medicament in the reservoir 1320 into nebulized particles upon input of an alternating voltage, for example. The nebulizer 1300 is connected to the mixing chamber 1100 via the aerosol port 1130, and is operable to provide the mixing chamber 1100 with a stream of nebulized particles 1135 from the nebulizer output port 1340. The aerosol port 1130 is of a diameter that is smaller than that of the body section 1110 of the mixing chamber 1100. This causes the flow velocity of nebulized particles 1135 to be reduced upon entering the settling volume.

In some embodiments, the nebulized particles 1135 are fed into an aerosol chamber 1350 prior to being introduced into the mixing chamber 1100. In some embodiments, the aerosol chamber 1350 is cylindrical and has a larger diameter than the diameter of the nebulizer outlet port 1340. This results in a reduction in flow velocity of the particles 1135 as they enter the aerosol chamber 1350. The aerosol chamber 1350 therefore disperses the particles 1135 into the mixing chamber 1110 at a lower speed. The aerosol chamber 1350 may be attached to the nebulizer outlet port 1340. In certain embodiments, the aerosol chamber 1350 may be contiguous with the nebulizer 1300. The diameter of the aerosol chamber 1350 may be marginally smaller than the diameter of the aerosol port 1130 of the mixing chamber 1100 thereby facilitating an interference fit between the aerosol port 1130 and the aerosol chamber 1350. In other embodiments, the aerosol port 1130 of the mixing chamber 1100 may have an internal thread that mates with an exterior thread formed on the aerosol chamber 1350. In some embodiments, the aerosol chamber 1350 may be provided with a baffle that is positioned within the aerosol chamber 1350 between the nebulizer output port 1340 and the point at which the aerosol port 1130 adjoins the mixing chamber 1100. The baffle enables the user to control the proportion of nebulized particles 1135 that is introduced into the mixing chamber 1100. In certain embodiments, the baffle may be adjustable to allow the user to adjust the rate at which the nebulized particles are introduced into the mixing chamber 1100.

The stream of nebulized particles 1135 enters the mixing chamber 1100 via the aerosol port 1130 and is mixed with the flow of breathing gas in the settling volume defined by the mixing chamber 1100 and the impacting cap 1200. Due to the aforementioned diameter of the body section 1110 of the mixing chamber 1100 in comparison with the diameter of the inlet port 1120 and the diameter of the aerosol port 1130, the flow velocity of the breathing gas and the flow velocity of the nebulized particles is reduced upon entry to the settling volume. Additionally, the flow of breathing gas supplied to the mixing chamber 1100 effectively 'pushes' the stream of nebulized particles 1135 upwards within the settling volume. This results in the nebulized particles having an upward velocity in the settling volume. As a result the nebulized particles 1135 move upwards at a reduced speed and impinge both the inner walls of the body section 1110 of the mixing chamber 1100 and the inner walls of the impacting cap 1200. This causes a portion of the nebulized particles to coalesce on the inner walls of the body section 1110 of the mixing chamber 1100 and the inner walls of the impacting cap 1200, thus resulting in rain-out. In some embodiments, due to the symmetrical configuration of sloped surfaces 1210, 1220 of the impacting cap 1200 about the vertical axis 1114 of the mixing chamber 1100, when the breathing gas impinges the impacting cap 1200, the breathing gas is directed downwards to the first opening 1142 of the outlet port 1140.

Due to gravity, the rain-out moves downwards along the inner walls of the impacting cap 1200 (as shown by arrow 1214 in FIG. 10) and along the inner walls of the body section 1110 of the mixing chamber 1100 (as shown by arrow 1114 in FIG. 9). The remaining nebulized particles are entrained with the breathing gas in the body section 1110 of the mixing chamber 1100 as they flow into the outlet port 1140 via the first end 1142 for delivery to the patient. In this manner, a portion of the nebulized particles is forced to coalesce and rain-out on the inner walls of both the body section 1110 of the mixing chamber 1100 and the sloped surfaces 1210, 1220 of the impacting cap 1200. This lowers the amount of nebulized medicament available in the mixing chamber 1100 for entrainment into the breathing gas at the first end 1142 of the outlet port 1140. In turn, this lowers the proportion of nebulized particles flowing through the delivery tube 1400 and thus reduces the possibility of further rain-out from occurring when the breathing gas, entrained with nebulized particles, is transported to the patient.

Mixing chamber 1100 may also comprise a collection chamber 1150 to collect any rain-out 1152 that forms in the settling volume. Collection chamber 1150 may be vertically oriented with respect to the mixing chamber 1100 such that it is positioned at the very bottom of the mixing chamber 1100. Collection chamber 1150 may also be located away from the first end 1142 of the outlet port 1140 to prevent re-entrainment of the rain-out back into the flow of heated and humidified gas flowing into the first opening 1142 of the outlet port 1140. The bottom of the collection chamber 1150 may additionally be fitted with a drainage port 1160 to facilitate removal of the rain-out from the mixing chamber 1100. Collection chamber 1150 may include a base that is angled or sloped so as to direct the rain-out towards the drainage port 1160. In certain embodiments, the collection chamber 1150 and the drainage port 1160 may be arranged such that they are positioned below the inlet port 1120, the first end 1142 of the outlet port 1140, and the aerosol port 1130. It will be understood that in order to prevent rain-out from entering the outlet port 1140, and to assist in the collection of rain-out in the collection chamber 1150, there are no horizontal surfaces or stepped edges near the first end 1142 of the outlet port 1140.

Drainage port 1160 may be fitted with a recirculation tube 1500 such that the rain-out 1152 is drained from the collection chamber 1150. A first end 1510 of the recirculation tube 1500 is attached to the drainage port 1160 and a second end 1520 of the recirculation tube 1500 is attached to the inlet 1310 of the nebulizer 1300. In this configuration, the rain-out 1152 that is drained from the collection chamber 1150 is dumped into the reservoir 1320 of the nebulizer 1300 via the inlet 1310. In this manner, the excess medicament that rains-out in the settling volume is recycled back to the reservoir 1320 of the nebulizer 1300. The rain-out is reintroduced into the reservoir 1320 to be nebulized as a stream of nebulized particles 1135 again. This prevents any wastage of medicament, and is particularly important for treatments involving expensive drugs.

The back pressure from the delivery tube 1400 and the nasal cannula assists with the recycling of the rained-out medicament. Here the back pressure acts on the rain-out 1152 in the collection chamber 1150 and is sufficient to push the rain-out 1152 through the drainage port 1160, along the length of the recirculation tube 1500 in the direction indicated by arrow 1530, and into the nebulizer reservoir 1320. In some embodiments, the recirculation tube 1500 may be dimensioned to have a diameter suitable to allow adequate flow of rain-out from the drainage port 1160 to the nebulizer 1300 while allowing only a small fraction of the breathing gas to enter into the recirculation tube 1500.

In certain embodiments, a flow restrictor may be attached to the drainage port 1160 to control the flowrate of the rain-out as it flows through the recirculation tube 1500 to the nebulizer 1300. Additionally, in further embodiments, the drainage port 1160 may be adapted with a siphon to drain some of the rain-out away from the recirculation tube 1500 and into an excess drug tank (not shown) for disposal. This would enable a user to drain any excess medicament from the system without having to disconnect the recirculation tube 1500. In some embodiments, the drainage port 1160 or the recirculation tube 1500 may be adapted with a stopcock to allow a user to stop the flow of the rain-out therethrough so as to allow the rain-out to be stored in the collection compartment 1150 of the mixing chamber 1100. Additionally, the recirculation tube 1500 may be provided with an auxiliary port (not shown) that is in fluid communication with the recirculating tube. The auxiliary port enables a user to inject additional or different medicament into the recirculation tube 1500 that may be needed during the course of respiratory therapy. This additional or different medicament gets dumped into the reservoir 1320 of the nebulizer 1300, which will subsequently become nebulized and delivered to the patient.

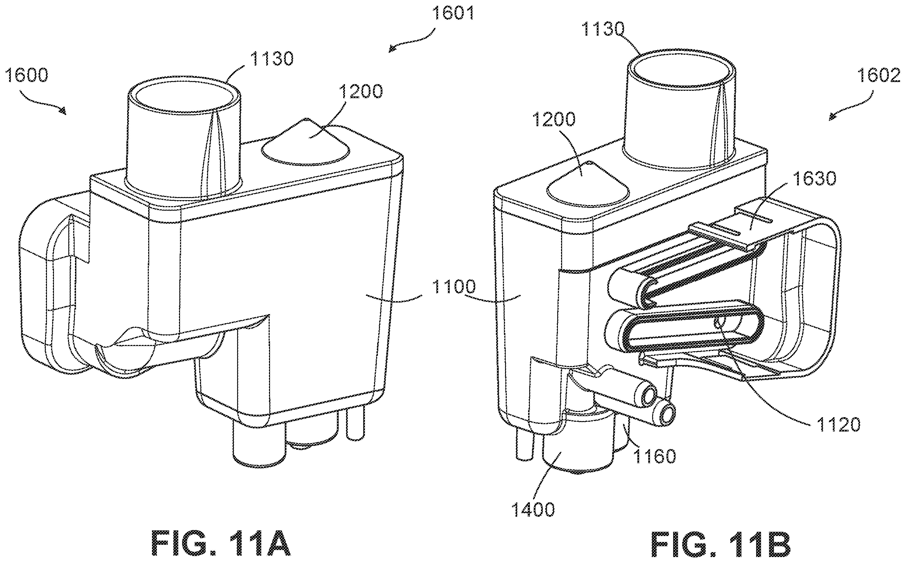

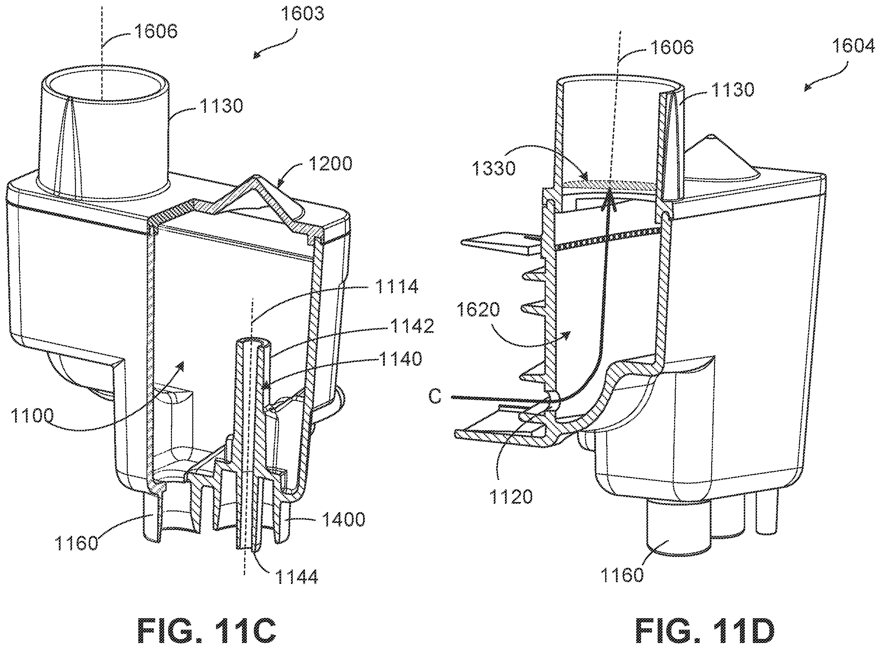

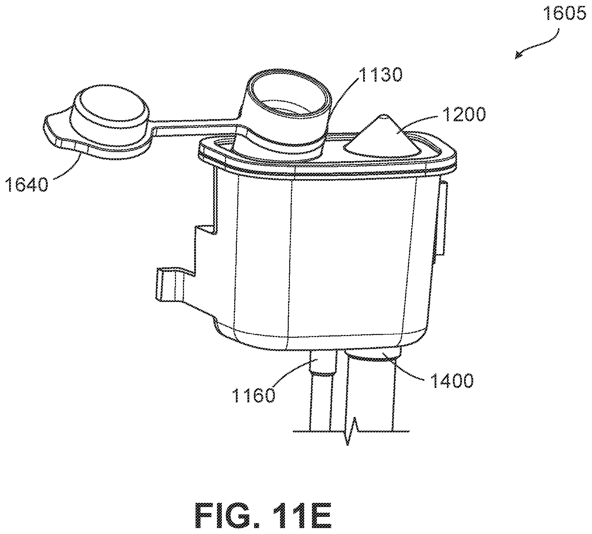

Vibrating mesh nebulizers, such as nebulizer 1300, also generate condensation (water droplets or droplets of medicament that has not been aerosolized) on the surface and/or at the edge of the vibrating mesh during operation. These droplets can slow or completely stop the aerosol production until the droplets fall off the surface of the mesh, or are shaken free. FIGS. 11A-11E illustrate several perspective views 1601-1605 of an exemplary embodiment of a nebulizer adaptor 1600 suitable for receiving a nebulizer, such as the vibrating mesh nebulizer 1300 as described in relation to FIGS. 9-10, for the provision of continuous aerosol therapy to a patient. The embodiments in FIGS. 11A-11E address the condensation issue associated with vibrating mesh nebulizers, while also providing continuous aerosol therapy to the patient.

FIGS. 11A-11E retains the features from the embodiments described in relation to FIGS. 9-10, as indicated by reference numbers in FIGS. 11A-11E, and thus the description of these similar features are omitted for brevity. Adaptor 1600 is similar to the body section 1110 of the mixing chamber 1100 as described in the foregoing, with the exception that the aerosol port 1130 is oriented such that its axis 1606 is substantially parallel to the axis 1114 of the mixing chamber 1100. In adaptor 1600, the inlet port 1120 is arranged orthogonally with respect to the axis 1606 of the aerosol port 1130. Adaptor 1600 forms a conduit 1620 between the inlet port 1120 and the aerosol port for the passage of breathing gas from a source of breathing gas (not shown). The passage of breathing gas is indicated by arrow C in FIG. 11D. The conduit 1620 is aligned with the axis 1606 of the aerosol port 1130. In some implementations, the breathing gas may be heated and humidified.

A vibrating mesh nebulizer is connected to the aerosol port 1130 of the adaptor 1600. The nebulizer is operable to provide a stream of nebulized particles to the adaptor 1600 for entrainment into the flow of breathing gas for delivery to the patient. Here the nebulized particles from a nebulizer connected to the aerosol port 1130 are entrained into flow C of breathing gas in conduit 1620, and the mixed stream is delivered to the settling volume of the mixing chamber 1100 for delivery to the patient via the outlet port 1140, in the manner as detailed with respect to FIGS. 9-10 as described above.

While not depicted in FIGS. 11A-11E, when the nebulizer is connected to the aerosol port 1130, the mesh 1330 of the nebulizer is positioned such that the axis 1606 of the aerosol port 1130 is normal to the surface of the mesh 1330 (mesh 1330 shown in FIG. 11D for reference). Due to the orientation of the conduit 1620, when the breathing gas flows in the conduit 1620 from inlet port 1120, the flow of the breathing gas is directed to impinge upon the surface of the mesh 1330 in a direction that is normal to the surface of the mesh 1330, as shown in FIG. 11D. In effect the direction of flow of breathing gas as it impinges the surface of mesh 1330 is aligned with the axis 1606 of the aerosol port 1130. The orientation of the flow of breathing gas with respect to the mesh 1330 of a nebulizer connected to the aerosol port 1130 enables the condensation that builds up on the surface and edges of the mesh 1330 to be removed. Such removal of condensate from the surface and edges of the mesh clears the mesh from any blockages, thereby improving the efficiency of the mesh in generating aerosolized medicament for entrainment with breathing gas within the adaptor 1600 for delivery to the patient. In certain implementations, the flowrate of breathing gas from the inlet 1120 is increased above the flowrate used for treatment of the patient when cleaning of the mesh 1330 is desired. The increased flowrate jets breathing gas at the nebulizer, at a direction normal to the surface of the mesh 1330, causing any droplets that may have developed during operation to be shaken off.

Adaptor 1600 also comprises attachment means, such as snap attachments 1630 on its outer surface, as shown in FIG. 11B. Such snap attachments 1630 enable the adaptor 1600 to be attached to a capital unit, such as the Precision Flow by Vapotherm, Inc. of Exeter, NH. This enables the adaptor 1600 to be compactly and rigidly attached to the body of such capital units and allows a supply of breathing gas to be fed into the inlet port 1120. In the case of the Precision Flow, the breathing gas may be heated and humidified prior to being fed into the inlet port 1120.

In some implementations, nebulizer adaptor 1600 may comprise a removable cap 1640 that is attached to the outer surface of the aerosol port 1130. When aerosol therapy for a patient has concluded, the mesh nebulizer is removed from the aerosol port 1130. In order to continue to provide breathing gas to the patient after aerosol therapy and without disconnecting the adaptor 1600, the cap 1640 is inserted into the aerosol port 1640. This seals the aerosol port 1640. Breathing gas from the inlet port 1120 then flows along conduit 1620 and into the settling volume of the mixing chamber 1100 for delivery to the patient via the outlet 1140. Thus the adaptor 1600 enables the continuous supply of breathing gas to the patient without interruption.

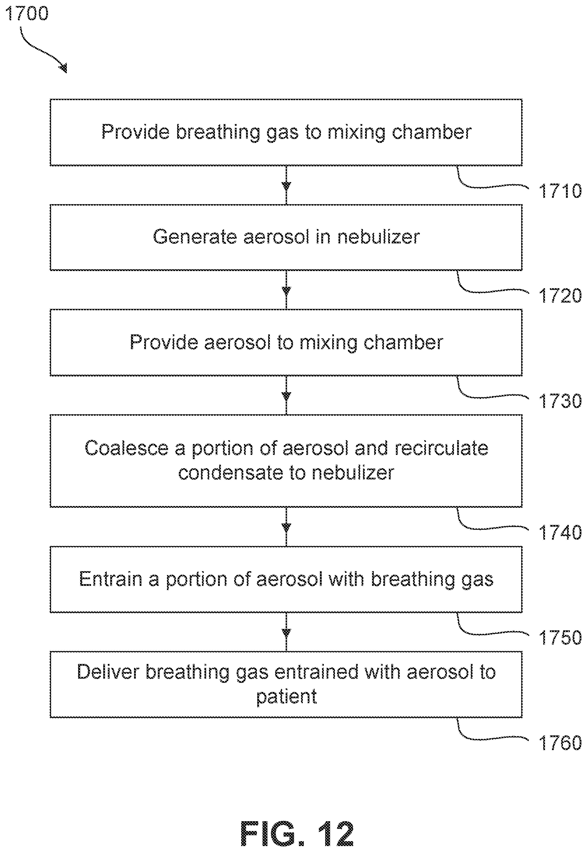

FIG. 12 shows a flowchart of an illustrative method 1700 for providing respiratory therapy to a patient. The method 1700 begins at step 1710 where a source of breathing gas is attached to a mixing chamber. The mixing chamber may be cylindrical. The breathing gas may be heated and humidified to reduce patient discomfort. The mixing chamber may have an input port, an outlet port, an aerosol port and a drainage port, and the source of breathing gas may be attached to the input port so as to provide the mixing chamber with a flow of breathing gas. The input port and the aerosol port may have a smaller diameter than the diameter of the mixing chamber. The mixing chamber may also comprise a collection compartment for collecting any condensate or rain-out that develops in the mixing chamber, the collection compartment being in fluid communication with the drainage port. Further, the mixing chamber may also have an impacting cap attached thereto, the impacting cap having at least one sloped surface. The mixing chamber and the impacting cap together form a settling volume. The setting volume may have a diameter that is larger than the diameter of the input port and the diameter of the aerosol port. Due to the geometry of the settling volume in relation to that of the input port, the velocity of the breathing gas may be reduced upon entry to the settling volume. Exemplary configurations of the mixing chamber have been described in relation to FIGS. 9, 10 and 11A-11E in the foregoing.

At step 1720, a stream of nebulized particles is generated by a nebulizer. The nebulizer may comprise a mesh having holes such that when the mesh is in a first state, liquid medicament stored in a reservoir is not permitted through the holes in the mesh, and when the mesh is in a second state, the liquid medicament is permitted to pass though the holes in the mesh. The nebulizer may comprise a piezoelectric ring that surrounds the mesh. The piezoelectric ring may be reactive to an input electric signal so as to cause a change of state of the mesh from the first state to the second state (or vice versa). When the electric signal is alternating in nature, such as an alternating voltage signal, for example, the mesh vibrates thereby generating a stream of aerosolized medicament.

At step 1730, the stream of nebulized particles is provided to the aerosol port of the mixing chamber. Due to the geometry of the settling volume in relation to that of the aerosol port, the velocity of the stream of nebulized particles may be reduced upon entry to the settling volume defined by the mixing chamber and the impacting cap. The stream of nebulized particles is mixed with the flow of breathing gas in the settling volume. The nebulized particles are 'pushed' upwards by the flow of breathing gas from the input port, imparting an upward velocity to the nebulized particles.

As a result, at step 1740, the nebulized particles move upwards within the mixing chamber and impinge the inner walls of the mixing chamber and the inner walls of the sloped surface of the impacting cap. This causes a portion of the nebulized particles to coalesce on the inner walls of the mixing chamber and the sloped surface of the impacting cap, resulting in rain-out in the mixing chamber. The rain-out moves downwards along the inner walls of the mixing chamber due to gravity and collects in the collection compartment. A recirculating tube is attached to the drainage port of the mixing chamber and connects the collection compartment to the reservoir in the nebulizer. The rain-out in the collection compartment may therefore be drained and recycled back into the reservoir to be reused.

At step 1750, the nebulized particles remaining in the mixing chamber are entrained with the breathing gas in the mixing chamber and flow into the outlet port. At step 1760, the breathing gas entrained with the remaining nebulized particles is delivered to the patient via a delivery tube connected to the outlet port of the mixing chamber.

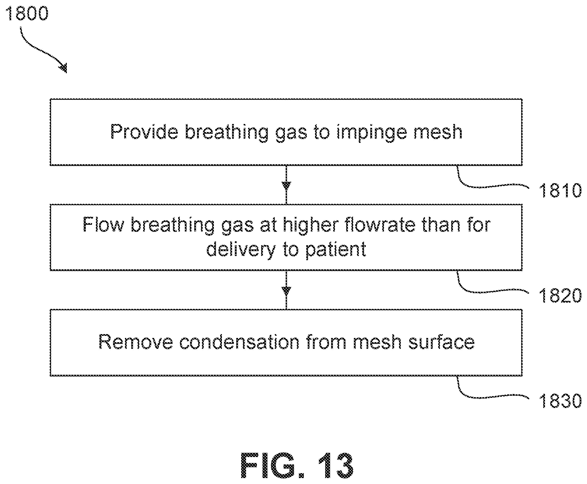

FIG. 13 shows a flowchart of an illustrative method 1800 for cleaning the vibrating mesh of a nebulizer attached to the aerosol port of a nebulizer adaptor, such as adaptor 1600 in FIGS. 11A-11E. The method 1800 begins at step 1810 where a flow of breathing gas is provided to the conduit 1620 from inlet port 1120. Here due to the orientation of the conduit 1620 with respect to the aerosol port 130m the flow of the breathing gas is directed to impinge upon the surface of the mesh 1330 in a direction that is normal to the surface of the mesh 1330. In step 1820, the flowrate of the breathing gas provided to the inlet port 1120 is increased above the flowrate used for delivery to the patient. The increased flowrate jets breathing gas at the nebulizer, at a direction normal to the surface of the mesh 1330, causing any condensation droplets that may have developed during operation of the vibrating mesh to be shaken off. This removes the condensation from the mesh (step 1830) thereby improving the efficiency of the mesh in generating aerosolized particles from a liquid medicament.

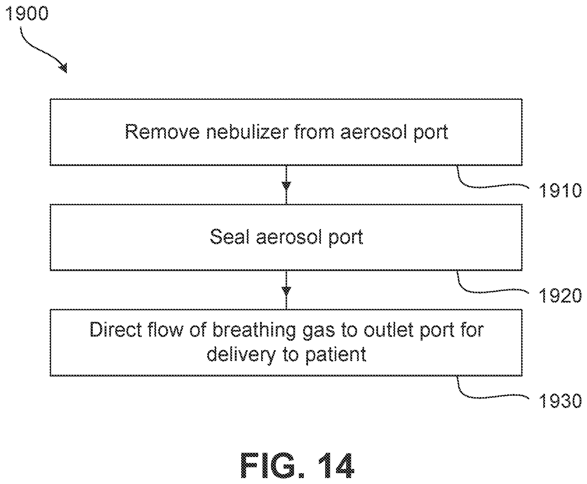

FIG. 14 shows a flowchart of an illustrative method 1900 for providing continuous respiratory therapy to a patient using a nebulizer adaptor, such as adaptor 1600 in FIGS. 11A-11E. The method 1900 begins at step 1910 where a nebulizer is removed from the aerosol port 1130 of the adaptor 1600, for example when aerosol therapy for a patient has concluded. A removable cap 1640 is then inserted into the aerosol port 1640 to seal the aerosol port 1640, as in step 1920. Breathing gas from the inlet port 1120 is then provided to conduit 1620 and directed into the settling volume of the mixing chamber 1100 for delivery to the patient via the outlet 1140, as in step 1930. This provides breathing gas to the patient after aerosol therapy and without having to disconnect the adaptor 1600, thereby enabling the continuous supply of breathing gas to the patient without interruption.

The foregoing is merely illustrative of the principles of the disclosure, and the apparatuses can be practiced by other than the described implementations, which are presented for purposes of illustration and not of limitation. It is to be understood that the apparatuses disclosed herein, while shown for use in high flow therapy systems, may be applied to systems to be used in other ventilation circuits.

Variations and modifications will occur to those of skill in the art after reviewing this disclosure. For example, while the inlet port 1120, the aerosol port 1130 and the drainage port 1160 are illustrated in FIGS. 9 and 10 as being arranged orthogonally to the mixing chamber 1100, it will be understood that some or all of these ports may be arranged at any angle with respect to the mixing chamber 1100 without deviating from the scope of the present disclosure. Further, while the nebulizer 1300 is illustrated in FIGS. 9 and 10 as being fixed at an angle with respect to the vertical axis 1114 of the mixing chamber 1100, the nebulizer 1300 may be oriented at any angle with respect to the axis 1114 of the mixing chamber 1100. Further, the nebulizer 1300 may be rotationally oriented at any angle with respect to the axis 1114 of the mixing chamber 1100.

It will be understood that respiratory medications such as bronchodilators, surfactants or antibiotics, may be administered, independently or in combination with each other, through inhalation directly to the patient's lungs using any of the embodiments disclosed in the foregoing. Bronchodilators include, but are not limited to, any medication for treating asthma or Chronic Obstructive Pulmonary Disease ("COPD"), such as Albuterol (Ventolin), Salbutamol (Proventil), and Levosalbutamol/ Levalbuterol (Xopenex), for example. Surfactants include, but are not limited to, any medication effective for treating diseases that alter the surface active properties of the lung, such as respiratory distress syndrome in premature infants ("iRDS"), acute respiratory distress syndrome (ARDS), asthma, pneumonia, acute lung injury (ALI), and meconium aspiration syndrome (MAS), for example. Surfactants for inhalation include, but are not limited to, Curosurf (Chiesi Pharmaceuticals), Alveofact (Boehringer Ingelheim), Survanta (Abbott Laboratories), Exosurf (Glaxo Wellcome), and Surfaxin (Discovery Laboratories), for example. Antibiotics include, but are not limited to, any antibiotics suitable for inhalation, such as macrolides (e.g., erythromycin, clarithromycin, azithromycin), glycopeptides (e.g. vancomycin and teicoplanin), oxazoldinone, quinupristin/dalfopristen, aminoglycosides (e.g., gentamicin, tobramycin, amikacin, streptomycin, netilmicin), quinolones (e.g., ciprofloxacin, ofloxacin, levofloxacin), tetracyclines (e.g., oxytetracycline, doxycycline, minocycline), cotrimoxazole, colistin, imepinim, and meripenim, for example. In some embodiments, any medication may be administered through inhalation directly to the patient's lungs using any of the embodiments disclosed in the foregoing.

The foregoing is merely illustrative of the principles of the disclosure, and the apparatuses can be practiced by other than the described implementations, which are presented for purposes of illustration and not of limitation. It is to be understood that the apparatuses disclosed herein, while shown for use in high flow therapy systems, may be applied to systems to be used in other ventilation circuits.

Variations and modifications will occur to those of skill in the art after reviewing this disclosure. The disclosed features may be implemented, in any combination and subcombination (including multiple dependent combinations and subcombinations), with one or more other features described herein. The various features described or illustrated above, including any components thereof, may be combined or integrated in other systems. Moreover, certain features may be omitted or not implemented.

Examples of changes, substitutions, and alterations are ascertainable by one skilled in the art and could be made without departing from the scope of the information disclosed herein. All references cited herein are incorporated by reference in their entirety and made part of this application.

The 43 claims of the parent application are reproduced immediately below as clauses.
Clause 1. An adaptor for introducing a nebulized medication into breathing gas for respiratory therapy, the adaptor comprising:
   a reservoir adapted to contain a volume of medication;
   a vibrating mesh disposed at a reservoir outlet of the reservoir and adapted to receive a flow of the medication from the reservoir and output a flow of nebulized medication;
   a mixing chamber having:
      a gas inlet adapted to receive a flow of breathing gas from a gas source;
      a nebulizer inlet adapted to receive the flow of nebulized medication from the vibrating mesh, the vibrating mesh being disposed between the nebulizer inlet and the reservoir outlet;
      an internal volume in fluid communication with the gas inlet and the nebulizer inlet and adapted to allow for mixing of the flow of breathing gas and the flow of nebulized medication to produced a flow of mixed gas; and
      a mixed gas outlet in fluid communication with the internal volume and adapted to output the flow of mixed gas; and
   a pressure tap tube in fluid communication with the mixing chamber via a first end of the pressure tap tube and the reservoir via a second end of the pressure tap tube, such that a gas in the pressure tap tube bypasses the nebulizer inlet and vibrating mesh to selectively equalize pressure between the mixing chamber and a headspace in the reservoir above the volume of medication.
Clause 2. The adaptor of clause 1, wherein the second end of the pressure tap tube comprises a connector sized to couple to a port of the reservoir in a sealingly manner and adapted to convey gas from the pressure tap tube into the headspace.
Clause 3. The adaptor of clause 2, wherein the connector is a rubber plug having an inner lumen.
Clause 4. The adaptor of any of clauses 2-3, further comprising a receiver sized to couple to the connector of the second end of the pressure tap tube, the receiver being isolated from the reservoir such that gas does not flow through the pressure tap tube when the connector is coupled to the receiver.
Clause 5. The adaptor of clause 1, wherein the reservoir comprises a valve adapted to couple to the second end of the pressure tap tube.
Clause 6. The adaptor of clause 5, wherein the valve is one of a solenoid valve, a diaphragm valve, or a needle valve.
Clause 7. The adaptor of clause 1, wherein the pressure tap tube is integrally formed in a wall connecting the reservoir to the mixing chamber, the wall at least partially defining the nebulizer inlet and positioned adjacent to the vibrating mesh.
Clause 8. The adaptor of any of clauses 1-7, wherein the reservoir comprises a medication inlet adapted to receive a refill flow of medication into the reservoir from a medication source.
Clause 9. The adaptor of clause 8, wherein the medication inlet comprises a sealing plug adapted.
Clause 10. The adaptor of any of clauses 1-9, wherein the pressure tap tube comprises a lumen having an internal diameter between 0.01 in and 0.03 in.
Clause 11. A method for providing breathing gas with nebulized medication at a high velocity, the method comprising:
   providing a flow of breathing gas at a first flow rate through a mixing chamber from a gas source;
   adjusting the flow of breathing gas to a second flow rate lower than the first flow rate;
   providing a flow of nebulized medication into the mixing chamber via a vibrating mesh;
   mixing the flow of nebulized medication with the flow of breathing gas at the second flow rate such that a flow of mixed gas is output from the mixing chamber;
   stopping the flow of nebulized medication by halting operation of the vibrating mesh; and
   adjusting the flow of breathing gas to the first flow rate.
Clause 12. The method of clause 11, wherein the breathing gas is provided at the second flow rate during a low flow period and at the first flow rate during a high flow period.
Clause 13. The method of clause 12, wherein the low flow period is synchronized with at least an inspiratory phase of breathing of a patient.
Clause 14. The method of any of clauses 12-13, wherein the high flow period is synchronized with an expiratory phase of breathing of a patient.
Clause 15. The method of any of clauses 13-14, wherein the low flow period occurs immediately before the inspiratory phase or immediately after the inspiratory phase.
Clause 16. The method of any of clauses 11-15, wherein the mixing chamber comprises a gas inlet having a first valve and a gas outlet having a second valve, wherein the first valve and second valve are closed while the flow of breathing gas is at the first flow rate, and wherein the first valve and second valve are open while the flow of breathing gas is at the second flow rate.
Clause 17. An adaptor for introducing a nebulized medication into breathing gas for respiratory therapy, the adaptor comprising:
   a gas inlet adapted to receive a flow of breathing gas from a gas source;
   a first flow path in fluid communication with the gas inlet and adapted to transmit a first portion of the flow of breathing gas;
   a second flow path in fluid communication with the gas inlet and adapted to transmit a second portion of the flow of breathing gas, the second flow path comprising a mixing chamber adapted to receive the second portion of breathing gas and a flow of nebulized medication, such that the second portion of breathing gas and the flow of nebulized medication mix to form a mixed portion of gas that is output by the second flow path;
   a reservoir adapted to hold a volume of medication;
   a vibrating mesh disposed between the reservoir and the mixing chamber and adapted to receive medication from the reservoir and output the flow of nebulized medication to a nebulizer inlet of the mixing chamber; and
   a gas outlet in fluid communication with the first flow path and the second flow path and adapted to combine the first portion of breathing gas and the mixed portion of gas to output a mixed flow of gas;
   wherein the first flow path and the second flow path are separated by a first valve, such the first valve may close to seal off the mixing chamber from the gas inlet.
Clause 18. The adaptor of clause 17, wherein when the flow of breathing gas is provided from the gas source at a high pressure the first valve passively closes to seal off the mixing chamber due to a pressure drop across the first valve generated by the high pressure.
Clause 19. The adaptor of clause 18, wherein when the first valve passively opens when the flow of breathing gas is adjusted to a low pressure.
Clause 20. The adaptor of clause 17, further comprising a controller operatively coupled to the first valve and the gas source and adapted to:
   control the gas source to adjust the flow of breathing gas from a first flow rate to a second flow rate, the second flow rate being higher than the first flow rate; and
   close the first valve so as to seal off the mixing chamber.
Clause 21. The adaptor of any of clauses 17-20, wherein the first valve is a diaphragm valve configured to occlude the second flow path in response to a high pressure flow of breathing gas.
Clause 22. The adaptor of any of clauses 17-21, wherein the first valve is positioned at a mixing chamber inlet, and further comprising a second valve positioned at a mixing chamber outlet, such that the mixing chamber is sealed off from the first flow path when the first valve and the second valve are closed.
Clause 23. A nebulizer comprising:
   a reservoir adapted to hold a volume of medication;
   a vibrating mesh in fluid communication with the reservoir and adapted to receive medication from the reservoir and output nebulized medication opposite the reservoir; and
   an aperture connecting the reservoir to the vibrating mesh, the aperture having a cross-section defined by a first length and a second length perpendicular to the first length, the second length being shorter than the first length.
Clause 24. The nebulizer of clause 23, wherein the aperture has an obround shape.
Clause 25. The nebulizer of any of clauses 23-24, wherein the cross-section of the aperture promotes air bubbles to move away from the vibrating mesh in a direction parallel to the first length.
Clause 26. A nebulizer comprising:
   a reservoir adapted to hold a volume of medication and having a medication outlet;
   a vibrating mesh disposed at the medication outlet and adapted to receive medication from the reservoir and output nebulized medication opposite the reservoir; and
   a pump having a pump outlet disposed within the reservoir for providing medication to the reservoir, the pump outlet being positioned proximally to the vibrating such that medication output from the pump outlet contacts the vibrating mesh.
Clause 27. The nebulizer of clause 26, wherein the pump is one of a syringe pump, a peristaltic pump, a diaphragm pump, a blower, or a bulb.
Clause 28. The nebulizer of any of clauses 26-27, wherein the pump outlet is at least partially contacting the vibrating mesh.
Clause 29. The nebulizer of any of clauses 26-28, wherein the pump delivers medication into the reservoir at a continuous rate.
Clause 30. The nebulizer of any of clauses 26-29, wherein the pump delivers medication into the reservoir in timed pulses.
Clause 31. A nebulizer comprising:
   a reservoir adapted to hold a volume of medication and having a medication outlet;
   a vibrating mesh disposed at the medication outlet and adapted to receive medication from the reservoir and output nebulized medication opposite the reservoir; and
   at least one of:
      a wicking material positioned within the reservoir and contacting the vibrating mesh, the wicking material being porous so as to draw medication towards to the vibrating mesh while allowing gas to pass through the wicking material away from the vibrating mesh;
      a protrusion extending into the reservoir proximal to the vibrating mesh; or a hydrophilic surface disposed on at least a portion of an inner surface of the reservoir.
Clause 32. A system for providing breathing gas and nebulized medication to a patient, the system comprising:
   a pressurized source of breathing gas adapted to provide a flow of breathing gas; an adaptor for introducing a nebulized medication into the breathing gas, the adaptor comprising:
   a reservoir adapted to contain a volume of medication;
   a vibrating mesh disposed at a reservoir outlet of the reservoir and adapted to receive a flow of the medication from the reservoir and output a flow of nebulized medication; and
   a mixing chamber having:
      a gas inlet coupled to the pressurized source of breathing gas and adapted to receive the flow of breathing gas from the pressurized source;
      a nebulizer inlet adapted to receive the flow of nebulized medication from the vibrating mesh, the vibrating mesh being disposed between the nebulizer inlet and the reservoir outlet;
      an internal volume in fluid communication with the gas inlet and the nebulizer inlet and adapted to allow for mixing of the flow of breathing gas and the flow of nebulized medication to produced a flow of mixed gas; and
      a mixed gas outlet in fluid communication with the internal volume and adapted to output the flow of mixed gas; and
   a pressure tap tube in fluid communication with the pressurized source and the reservoir, such that a gas in the pressure tap tube bypasses the mixing chamber to pressurize a headspace in the reservoir above the volume of medication.
Clause 33. The system of clause 32, wherein the pressure tap tube comprises a first end coupled to the pressurized source and a second end coupled to the reservoir.
Clause 34. The system of clause 33, wherein the second end is part of a port adapted to also provide a refill flow of medication into the reservoir from a medication source.
Clause 35. The system of any of clauses 32-34, further comprising a controller operatively coupled to the pressurized source to control a flow rate of the flow of breathing gas and to the vibrating mesh to control the output of nebulized medication.
Clause 36. The system of clause 35, wherein the pressure tap tube comprises a valve operatively coupled to the controller, such that the controller is configured to control a flow rate of gas through the pressure tap tube.
Clause 37. The system of clause 36, wherein the controller is configured to automatically adjust the flow rate of gas through the pressure tap tube based on the flow rate of breathing gas from the pressurized source in order to control a pressure drop across the vibrating mesh to reach a target pressure drop.
Clause 38. The system of any of clauses 32-37, further comprising a nasal cannula in fluid communication with the mixed gas outlet and configured to deliver the flow of mixed gas to a patient.
Clause 39. A system for providing breathing gas and nebulized medication to a patient, the system comprising:
   a pressurized source of breathing gas adapted to provide a flow of breathing gas;
   an adaptor for introducing a nebulized medication into the breathing gas, the adaptor comprising:
      a reservoir adapted to contain a volume of medication;
   a vibrating mesh disposed at a reservoir outlet of the reservoir and adapted to receive a flow of the medication from the reservoir and output a flow of nebulized medication; and
   a mixing chamber having:
      a gas inlet in fluid communication with the pressurized source of breathing gas and adapted to receive the flow of breathing gas from the pressurized source;
      a nebulizer inlet adapted to receive the flow of nebulized medication from the vibrating mesh, the vibrating mesh being disposed between the nebulizer inlet and the reservoir outlet;
      an internal volume in fluid communication with the gas inlet and the nebulizer inlet and adapted to allow for mixing of the flow of breathing gas and the flow of nebulized medication to produced a flow of mixed gas; and
      a mixed gas outlet in fluid communication with the internal volume and adapted to output the flow of mixed gas; and
   a pressure tap tube having a first end in fluid communication with the reservoir, such that a gas in the pressure tap tube pressurizes a headspace in the reservoir above the volume of medication.
Clause 40. The system of clause 39, wherein the pressure tap tube has a second end in fluid communication with the pressurized source such that pressurized gas from the pressurized source is transmitted to the headspace.
Clause 41. The system of clause 39, further comprising a gas tube coupling the pressurized source to the gas inlet of the mixing chamber, and wherein the pressure tap tube has a second end in fluid communication with one of the delivery tube or the mixing chamber, such that a portion of breathing gas is diverted from the delivery tube or mixing chamber to pressurize the headspace.
Clause 42. The adaptor, method, or system of any of clauses 1-22 and 32-40, wherein the breathing gas is humidified.
Clause 43. The adaptor, method, or system of any of clauses 1-22 and 32-40, wherein the breathing gas is provided at a flow rate between 8 LPM and 60 LPM.

## Claims

1. An adaptor for introducing a nebulized medication into breathing gas for respiratory therapy, the adaptor comprising:
a reservoir adapted to contain a volume of medication;
a vibrating mesh disposed at a reservoir outlet of the reservoir and adapted to receive a flow of the medication from the reservoir and output a flow of nebulized medication;
a mixing chamber having:
a gas inlet adapted to receive a flow of breathing gas;
a nebulizer inlet adapted to receive the flow of nebulized medication from the vibrating mesh, the vibrating mesh being disposed between the nebulizer inlet and the reservoir outlet;
an internal volume in fluid communication with the gas inlet and the nebulizer inlet and adapted to allow for mixing of the flow of breathing gas and the flow of nebulized medication to produced a flow of mixed gas;
a mixed gas outlet in fluid communication with the internal volume and adapted to output the flow of mixed gas; and
a pressure tap tube in fluid communication with the mixing chamber via a first end of the pressure tap tube and the reservoir via a second end of the pressure tap tube, such that a gas in the pressure tap tube bypasses the nebulizer inlet and vibrating mesh to selectively equalize pressure between the mixing chamber and a headspace in the reservoir above the volume of medication;
wherein the pressure tap tube is integrally formed in a wall connecting the reservoir to the mixing chamber, the wall at least partially defining the nebulizer inlet and positioned adjacent to the vibrating mesh.

2. The adaptor of claim 1, wherein the reservoir comprises a medication inlet adapted to receive a flow of medication into the reservoir from a medication source.

3. The adaptor of claim 2, wherein the medication inlet comprises a sealing plug adapted.

4. The adaptor of any of claims 1-3, wherein the pressure tap tube comprises a lumen having an internal diameter between 0.25mm and 0.76mm.

5. The adaptor of claim 1, further comprising a controller connected to the vibrating mesh to control output of the nebulized medication.

6. The adaptor of claim 5, wherein the controller is configured to control a pressure drop across the vibrating mesh by automatically adjusting a flow rate of the breathing gas through the pressure tap tube based on a flow rate of the breathing gas received by the gas inlet.

7. The adaptor of claim 6, further comprising one or more of:
a pressure sensor;
a flow velocity sensor;
a flow rate sensor; and
a temperature sensor;
wherein the controller is configured to control a pressure drop across the vibrating mesh by automatically adjusting the flow rate of the breathing gas through the pressure tap tube based on one or more measurements obtained from the one or more sensors.

8. The adaptor of any of claims 5-7, wherein the controller is configured to be operatively coupled to a source of the breathing gas received by the gas inlet and to control a flow rate of the breathing gas.

9. The adaptor of claim 8, comprising a breathing sensor, wherein the controller is configured to provide a reduction in the flow rate of the breathing gas received by the gas inlet, said reduction being synchronized with at least an inspiratory breathing phase.

10. The adaptor of claim 9, wherein the controller is configured to provide the reduction in the first flow rate of the breathing gas at the gas inlet immediately before the inspiratory phase or immediately after the inspiratory phase.

11. A system for providing breathing gas and nebulized medication to a patient, the system comprising:
an adaptor according to any of the preceding claims; and
a pressurized source gas adapted to provide the flow of breathing gas to the gas inlet.

12. The system of claim 11, wherein the breathing gas is humidified.

13. The system of claim 11 or 12, further comprising a nasal cannula in fluid communication with the mixed gas outlet and configured to receive the flow of mixed gas.

14. The system of any of claims 11-13, wherein the gas source is configured to provide the first and second flows of the breathing gas a flow rate between 8 LPM and 60 LPM.
